(19) Europäisches Patentamt
European Patent Office
Office européen des brevets

(11) EP 1 056 342 B1

(12) EUROPÄISCHE PATENTSCHRIFT

(45) Veröffentlichungstag und Bekanntmachung des Hinweises auf die Patenterteilung:
**20.08.2003 Patentblatt 2003/34**

(21) Anmeldenummer: **99904877.0**

(22) Anmeldetag: **10.02.1999**

(51) Int Cl.$^7$: **A01N 51/00**, A01N 61/00
// (A01N51/00, 43:08, 31:02), A01N25:02,(A01N61/00, 43:08, 31:02), A01N25:02

(86) Internationale Anmeldenummer:
**PCT/EP99/00878**

(87) Internationale Veröffentlichungsnummer:
**WO 99/041987 (26.08.1999 Gazette 1999/34)**

(54) **WASSERHALTIGE MITTEL ZUR BEKÄMPFUNG PARASITIERENDER INSEKTEN UND MILBEN AN MENSCHEN**

AQUEOUS AGENTS FOR COMBATING PARASITIC INSECTS AND ACARINA IN HUMAN BEINGS

PRODUITS AQUEUX POUR LA LUTTE CONTRE LES INSECTES PARASITES ET LES ACARIENS CHEZ L'HOMME

(84) Benannte Vertragsstaaten:
**AT BE CH CY DE DK ES FI FR GB GR IE IT LI LU MC NL PT SE**

(30) Priorität: **23.02.1998 DE 19807630**

(43) Veröffentlichungstag der Anmeldung:
**06.12.2000 Patentblatt 2000/49**

(73) Patentinhaber: **Bayer Aktiengesellschaft**
**51368 Leverkusen (DE)**

(72) Erfinder:
- **SIRINYAN, Kirkor**
**D-51467 Bergisch Gladbach (DE)**
- **HORN, Karin**
**D-42679 Soligen (DE)**
- **STÖCKER, Ronald, Helmut**
**D-40789 Monheim (DE)**
- **SONNECK, Rainer**
**D-51375 Leverkusen (DE)**

(56) Entgegenhaltungen:
**DE-A- 4 443 888**
**DE-A- 19 540 948**
**DE-A- 19 543 477**
**DE-A- 19 613 334**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 1 056 342 B1

## Beschreibung

Die vorliegende Erfindung betrifft wasserhaltige Formulierungen zur dermalen Bekämpfung von parasitierenden Insekten und Milben an Menschen mittels Agonisten oder Antagonisten der nicotinergen Acetylcholinrezeptoren von Insekten.

Agonisten oder Antagonisten der nicotinergen Acetylcholinrezeptoren von Insekten sind bekannt. Zu ihnen gehören die Nicotinyl-Insektizide und ganz besonders die Chlornicotinyl-Insektizide.

Aus DE-A-19 613 334 sind Formulierungen bekannt zur dermalen Anwendung von Agonisten oder Antagonisten der nicotinergen Acetylcholinrezeptoren, die sich zur Bekämpfung parasitierender Insekten und Milben an Menschen eignen. Diese Formulierungen basieren auf Mischungen organischer Lösungsmittel.

Es wurden nun neue wasserhaltigen Formulierungen zur dermalen Anwendung von Agonisten oder Antagonisten der nicotinergen Acetylcholinrezeptoren, die sich zur Bekämpfung parasitierender Insekten und Milben eignen, der folgenden Zusammensetzung gefunden:

a - Agonisten oder Antagonisten der nicotinergen Acetylcholinrezeptoren von Insekten in einer Konzentration von 0,0001 bis 7,5 Gew.-%, bezogen auf das Gesamtgewicht der Formulierung;

b - Wasser in einer Konzentration von 20 bis 50 Gew.-%, bezogen auf das Gesamtgewicht der Formulierung;

c - acyclische Alkohole in einer Konzentration von 20 bis 50 Gew.-%, bezogen auf das Gesamtgewicht der Formulierung;

d - cyclische Carbonate in einer Konzentration von 2,5 bis zu 20,0 Gew.-%, bezogen auf das Gesamtgewicht der Formulierung;

e - gegebenenfalls weitere Hilfsmittel aus der Gruppe Verdickungsmittel, Spreitmittel, Farbstoffe, Antioxidantien, Treibmittel, Konservierungsstoffe, Haftmittel, Emulgatoren, in einer Konzentration von 0 bis zu 30 Gew.-%, bezogen auf das Gesamtgewicht der Formulierung.

Agonisten oder Antanogisten der nicotinergen Acetylcholinrezeptoren von Insekten sind bekannt z.B. aus Europäische Offenlegungsschriften Nr. 580 553, 464 830, 428 941, 425 978, 386 565, 383 091, 375 907, 364 844, 315 826, 259 738, 254 859, 235 725, 212 600, 192 060, 163 855, 154 178, 136 636, 303 570, 302 833, 306 696, 189 972, 455 000, 135 956, 471 372, 302 389; Deutsche Offenlegungsschriften Nr. 3 639 877, 3 712 307; Japanische Offenlegungsschriften Nr. 03 220 176, 02 207 083, 63 307 857, 63 287 764, 03 246 283, 04 9371, 03 279 359, 03 255 072; US-Patentschriften Nr. 5 034 524, 4 948 798, 4 918 086, 5 039 686, 5 034 404; PCT-Anmeldungen Nr. WO 91/17 659, 91/4965; Französische Anmeldung Nr. 2 611 114; Brasilianische Anmeldung Nr. 88 03 621.

Auf die in diesen Publikationen beschriebenen Verbindungen und ihre Herstellung wird hiermit ausdrücklich Bezug genommen.

Diese Verbindungen lassen sich bevorzugt durch die allgemeine Formel (I) wiedergeben

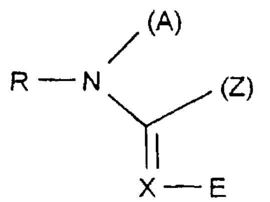

(I),

in welcher

R für Wasserstoff, gegebenenfalls substituierte Reste der Gruppe Acyl, Alkyl, Aryl, Aralkyl, Heteroaryl oder Heteroarylalkyl steht;

A für eine monofunktionelle Gruppe aus der Reihe Wasserstoff, Acyl, Alkyl, Aryl steht oder für eine bifunktionelle Gruppe steht, die mit dem Rest Z verknüpft ist;

E für einen elektronenziehenden Rest steht;

X für die Reste -CH= oder =N- steht, wobei der Rest -CH= anstelle eines H-Atoms mit dem Rest Z verknüpft sein kann;

Z für eine monofunktionelle Gruppe aus der Reihe Alkyl, -O-R, -S-R,

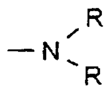

steht
oder für eine bifunktionelle Gruppe steht, die mit dem Rest A oder dem Rest X verknüpft ist.

Besonders bevorzugt sind Verbindungen der Formel (I), in welcher die Reste folgende Bedeutung haben:

R steht für Wasserstoff sowie für gegebenenfalls substituierte Reste aus der Reihe Acyl, Alkyl, Aryl, Aralkyl, Heteroaryl, Heteroarylalkyl, Heterocyclylalkyl.
Als Acylreste seien genannt Formyl, Alkylcarbonyl, Arylcarbonyl, Alkylsulfonyl, Arylsulfonyl, (Alkyl-)-(Aryl-)-phosphoryl, die ihrerseits substituiert sein können.
Als Alkyl seien genannt $C_{1-10}$-Alkyl, insbesondere $C_{1-4}$-Alkyl, im einzelnen Methyl, Ethyl, i-Propyl, sec.- oder t.-Butyl, die ihrerseits substituiert sein können.
Als Aryl seien genannt Phenyl, Naphthyl, insbesondere Phenyl.
Als Aralkyl seien genannt Phenylmethyl, Phenylethyl.
Als Heteroaryl seien genannt Heteroaryl mit bis zu 10 Ringatomen und N, O oder S insbesondere N als Heteroatomen. Im einzelnen seien genannt Thienyl, Furyl, Thiazolyl, Imidazolyl, Pyridyl, Benzthiazolyl.
Als Heteroarylalkyl seien genannt Heteroarylmethyl, Heteroarylethyl, wobei Heteroaryl bevorzugt bis zu 6 Ringatome und N, O oder S, insbesondere N als Heteroatome enthält. Besonders bevorzugt seien die oben angegebenen Heteroarylreste genannt.
Als Heterocyclylalkyl sei genannt Tetrahydrofuranylmethyl.
Als Substituenten seien beispielhaft und vorzugsweise aufgeführt:

Alkyl mit vorzugsweise 1 bis 4, insbesondere 1 oder 2 Kohlenstoffatomen, wie Methyl, Ethyl, n- und i-Propyl und n-, i- und t-Butyl; Alkoxy mit vorzugsweise 1 bis 4, insbesondere 1 oder 2 Kohlenstoffatomen, wie Methoxy, Ethoxy, n- und i-Propyloxy und n-, i- und t-Butyloxy; Alkylthio mit vorzugsweise 1 bis 4, insbesondere 1 oder 2 Kohlenstoffatomen, wie Methylthio, Ethylthio, n- und i-Propylthio und n-, i- und t-Butylthio; Halogenalkyl mit vorzugsweise 1 bis 4, insbesondere 1 oder 2 Kohlenstoffatomen und vorzugsweise 1 bis 5, insbesondere 1 bis 3 Halogenatomen, wobei die Halogenatome gleich oder verschieden sind und als Halogenatome, vorzugsweise Fluor, Chlor oder Brom, insbesondere Fluor stehen, wie Trifluormethyl; Hydroxy; Halogen, vorzugsweise Fluor, Chlor, Brom und Jod, insbesondere Fluor, Chlor und Brom; Cyano; Nitro; Amino; Monoalkyl- und Dialkylamino mit vorzugsweise 1 bis 4, insbesondere 1 oder 2 Kohlenstoffatomen je Alkylgruppe, wie Methylamino, Methyl-ethyl-amino, n- und i-Propylamino und Methyl-n-butylamino; Carboxyl; Carbalkoxy mit vorzugsweise 2 bis 4, insbesondere 2 oder 3 Kohlenstoffatomen, wie Carbomethoxy und Carboethoxy; Sulfo ($-SO_3H$); Alkylsulfonyl mit vorzugsweise 1 bis 4, insbesondere 1 oder 2 Kohlenstoffatomen, wie Methylsulfonyl und Ethylsulfonyl; Arylsulfonyl mit vorzugsweise 6 oder 10 Arylkohlenstoffatomen, wie Phenylsulfonyl sowie Heteroarylamino und Heteroarylalkylamino wie Chlorpyridylamino und Chlorpyridylmethylamino.

A steht besonders bevorzugt für Wasserstoff sowie für gegebenenfalls substituierte Reste aus der Reihe Acyl, Alkyl, Aryl, die bevorzugt die bei R angegebenen Bedeutungen haben. A steht ferner für eine bifunktionelle Gruppe. Genannt sei gegebenenfalls substituiertes Alkylen mit 1-4, insbesondere 1-2 C-Atomen, wobei als Substituenten die weiter oben aufgezählten Substituenten genannt seien und wobei die Alkylengruppen durch Heteroatome aus der Reihe N, O oder S unterbrochen sein können.

A und Z können gemeinsam mit den Atomen, an welche sie gebunden sind, einen gesättigten oder ungesättigten heterocyclischen Ring bilden. Der heterocyclische Ring kann weitere 1 oder 2 gleiche oder verschiedene Heteroatome und/oder Heterogruppen enthalten. Als Heteroatome stehen vorzugsweise Sauerstoff, Schwefel oder Stickstoff und als Heterogruppen N-Alkyl, wobei Alkyl der N-Alkyl-Gruppe vorzugsweise 1

bis 4, insbesondere 1 oder 2 Kohlenstoffatome enthält. Als Alkyl seien Methyl, Ethyl, n- und i-Propyl und n-, i- und t-Butyl genannt. Der heterocyclische Ring enthält 5 bis 7, vorzugsweise 5 oder 6 Ringglieder.
Als Beispiele für den heterocyclischen Ring seien Pyrrolidin, Piperidin, Piperazin, Hexamethylenimin, Hexahydro-1,3,5-triazin, Morpholin, Oxadiazin genannt, die gegebenenfalls bevorzugt durch Methyl substituiert sein können.

E steht für einen elektronenziehenden Rest, wobei insbesondere $NO_2$, CN, Halogenalkylcarbonyl wie 1-5-Halogen-$C_{1-4}$-carbonyl, insbesondere $COCF_3$ sowie Alkylsuslfonyl und Halogenalkylsulfonyl wie 1-5-Halogen-$C_1$-$C_4$-sulfonyl, insbesondere $SO_2CF_3$, genannt seien.

X steht für -CH= oder -N= .

Z steht für gegebenenfalls substituierte Reste Alkyl, -OR, -SR, -NRR (die Reste R sind gleich oder verschieden), wobei R und die Substituenten bevorzugt die oben angegebene Bedeutung haben.

Z kann außer dem obengenannten Ring gemeinsam mit dem Atom, an welches es gebunden ist und dem Rest

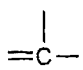

an der Stelle von X einen gesättigten oder ungesättigten heterocyclischen Ring bilden. Der heterocyclische Ring kann weitere 1 oder 2 gleiche oder verschiedene Heteroatome und/oder Heterogruppen enthalten. Als Heteroatome stehen vorzugsweise Sauerstoff, Schwefel oder Stickstoff und als Heterogruppen N-Alkyl, wobei die Alkyl oder N-Alkyl-Gruppe vorzugsweise 1 bis 4, insbesondere 1 oder 2 Kohlenstoffatome enthält. Als Alkyl seien Methyl, Ethyl, n- und i-Propyl und n-, i- und t-Butyl genannt. Der heterocyclische Ring enthält 5 bis 7, vorzugsweise 5 oder 6 Ringglieder.
Als Beispiele für den heterocyclischen Ring seien Pyrrolidin, Piperidin, Piperazin, Hexamethylenimin, Morpholin und N-Methylpiperazin genannt.

Besonders erwähnt sei außerdem die Verwendung von Verbindungen der Formel (I), die dadurch gekennzeichnet sind, daß die Reste in Formel (I) folgende Bedeutung haben:

R steht für gegebenenfalls substituierte Reste aus der Reihe Heteroarylmethyl oder Heteroarylethyl, wobei als Heteroaryl genannt sei:

Tienyl, Furyl, Thiazolyl, Imidazolyl, Pyridyl, Benzthiazolyl.

Als Substituenten seien aufgeführt:

Methyl, Ethyl, Methoxy, Ethoxy, Methylthio, Ethylthio, Trifluormethyl; Hydroxy; Fluor, Chlor und Brom; Cyano; Nitro; Amino;

A steht für Wasserstoff sowie für eine mit dem Rest Z verknüpfte bifunktionelle, gegebenenfalls substituierte Alkylengruppe mit 2 C-Atomen, wobei als Substituenten die weiter oben aufgezählten Substituenten genannt seien und wobei die Alkylengruppe durch 1 Heteroatom aus der Reihe N, O oder S unterbrochen sein kann,

A und Z können gemeinsam mit den Atomen, an welche sie gebunden sind, einen gesättigten oder ungesättigten 5- oder 6-gliedrigen heterocyclischen Ring bilden. Der heterocyclische Ring kann weitere 1 oder 2 gleiche oder verschiedene Heteroatome und/oder Heterogruppen enthalten. Als Heteroatome stehen Sauerstoff, Schwefel oder Stickstoff und als Heterogruppen N-Alkyl, wobei Alkyl der N-Alkyl-Gruppe 1 oder 2 Kohlenstoffatome enthält.

E steht für $NO_2$, CN.

X steht für -CH= oder -N= .

Z steht für gegebenenfalls substituierte Reste Alkyl, -OR',-SR', -NR'R', (die Reste R' sind gleich oder verschieden) wobei R' und die Substituenten folgende Bedeutung haben:

R' steht für Wasserstoff sowie für gegebenenfalls substituierte Reste aus der Reihe Acyl, Alkyl, Aryl, Aralkyl, Heteroaryl, Heteroarylalkyl.
Als Acylreste seien genannt Formyl, Alkylcarbonyl, Arylcarbonyl, Alkylsulfonyl, Arylsulfonyl, (Alkyl-)-(Aryl-)-phosphoryl.
Als Alkyl sei $C_{1-4}$-Alkyl genannt.
Als Aryl sei Phenyl genannt.
Als Aralkyl seien genannt Phenylmethyl, Phenylethyl.
Als Heteroarylalkyl seien genannt Heteroarylmethyl, Heteroarylethyl, wobei als Heteroaryl genannt sei Thienyl, Furyl, Thiazolyl, Imidazolyl, Pyridyl, Benzthiazolyl.
Als Substituenten der Reste R' seien aufgeführt:

Methyl, Ethyl, Methoxy, Ethoxy, Methylthio, Ethylthio, Halogenalkyl mit 1 oder 2 Kohlenstoffatomen und 1 bis 5 Halogenatomen, wobei die Halogenatome gleich oder verschieden sind und als Halogenatome Fluor, Chlor oder Brom stehen, Hydroxy; Fluor, Chlor und Brom; Cyano, Nitro, Amino, Mono- alkyl- und Dialkylamino mit vorzugsweise 1 oder 2 Kohlenstoffatomen je Alkylgruppe, Carboxyl; Carbalkoxy mit 2 oder 3 Kohlenstoffatomen, Sulfo ($-SO_3H$); Alkylsulfonyl mit 1 oder 2 Kohlenstoffatomen, Phenylsulfonyl Chlorpyridylamino und Chlorpyridylmethylamino.

Als ganz besonders bevorzugt erfindungsgemäß verwendbare Verbindungen seien Verbindungen der allgemeinen Formeln (II), (III) und (IV) genannt:

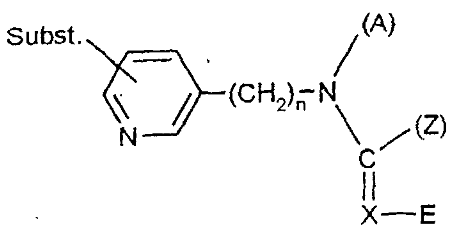

(II),

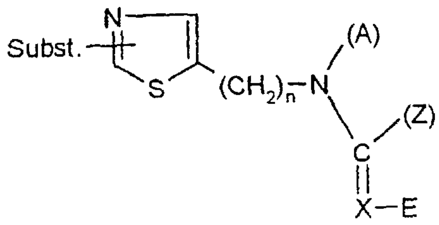

(III),

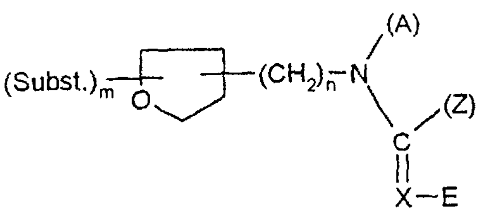

(IV),

in welchen

n für 1 oder 2 steht,

m für 0, 1 oder 2 steht,

Subst. für einen der oben aufgeführten Substituenten, insbesonders für Halogen, ganz besonders für Chlor, steht, A, Z, X und E die oben angegebenen Bedeutungen haben.

Im einzelnen seien folgende Verbindungen genannt:

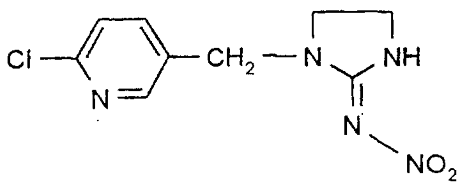

Imidacloprid

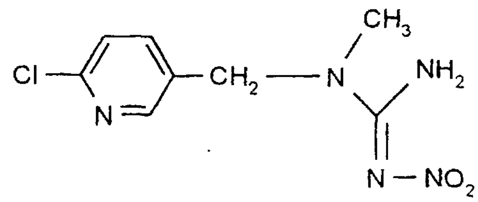

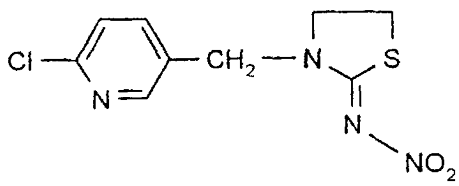

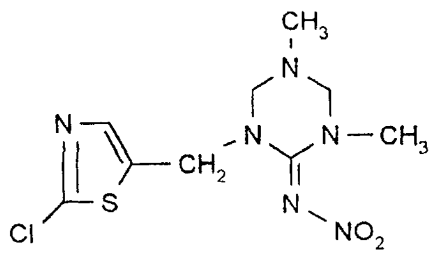

AKD 1022

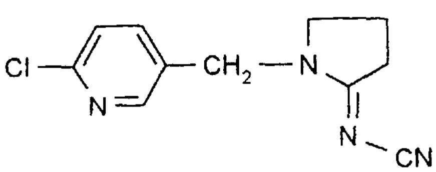

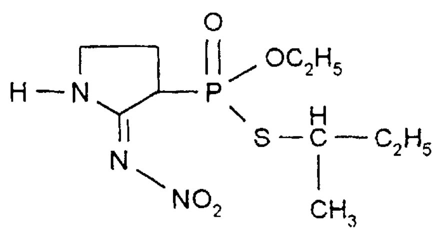

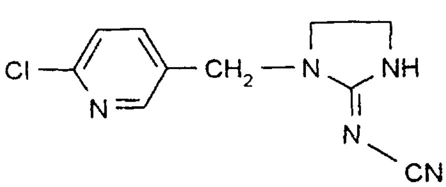

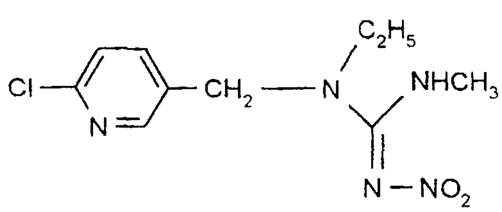

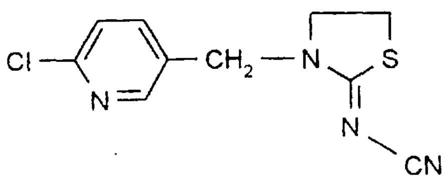

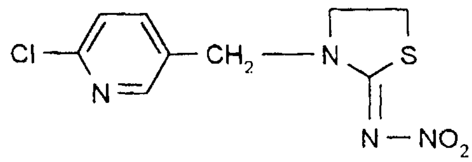

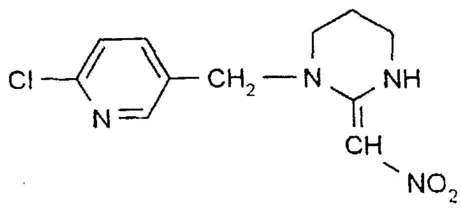

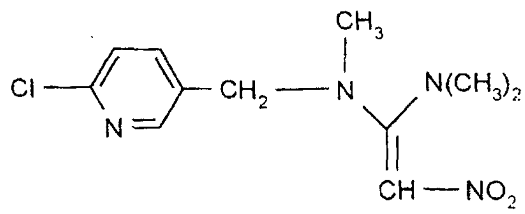

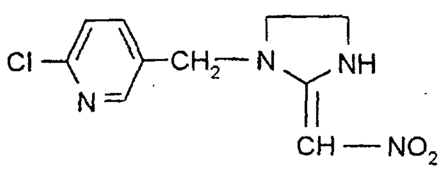

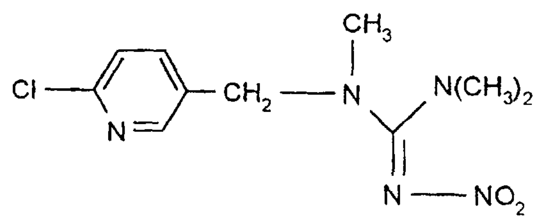

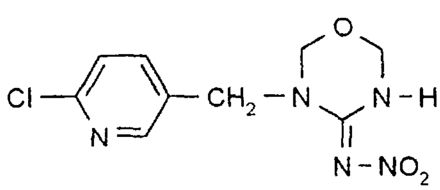

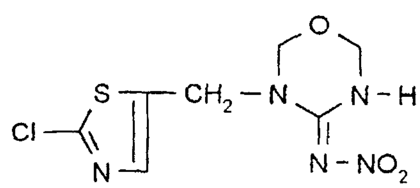

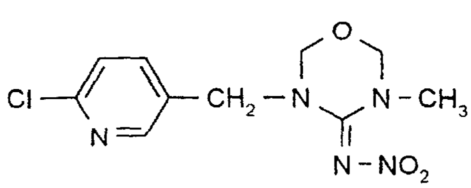

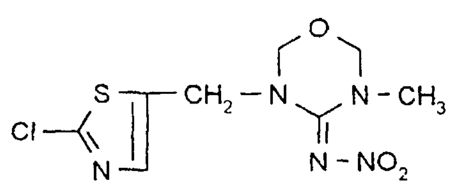

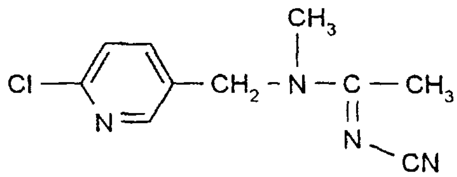

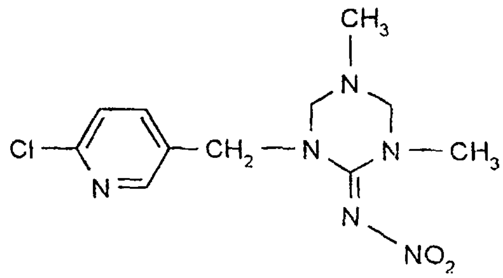

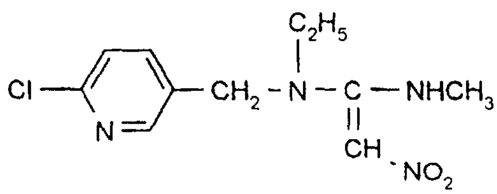

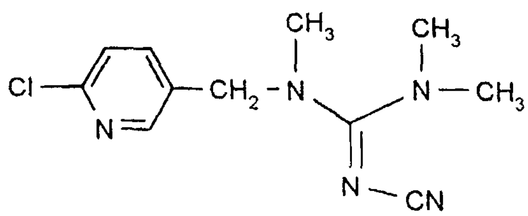

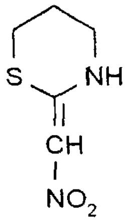

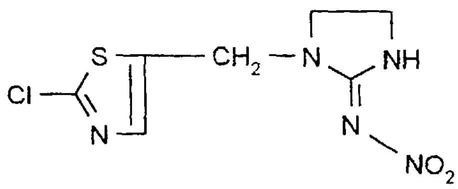

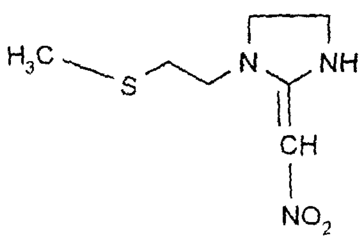

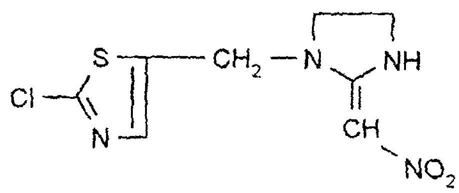

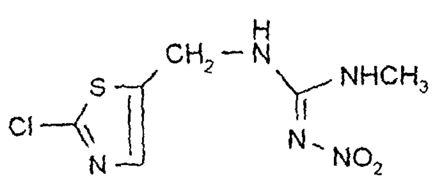

Ti435

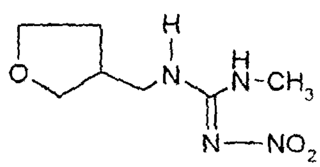

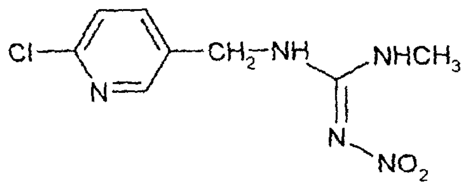

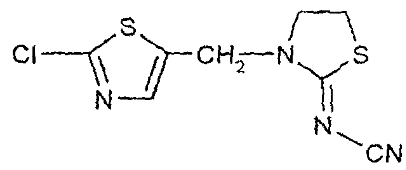

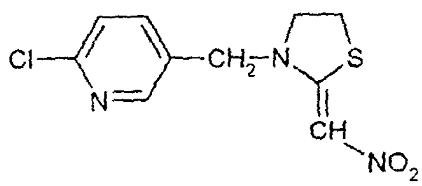

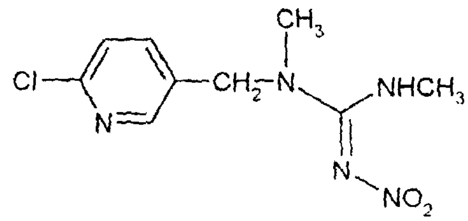

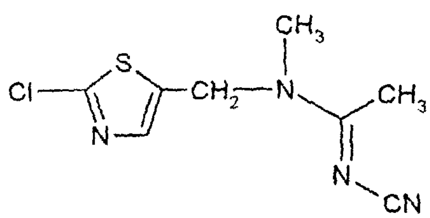

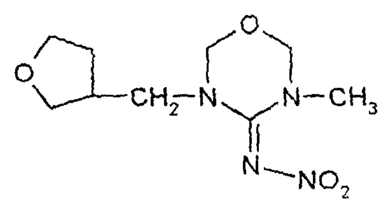

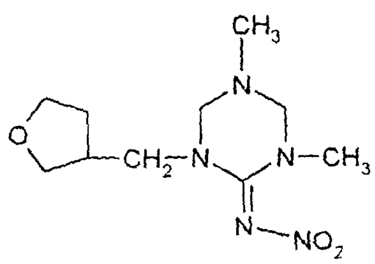

Besonders hervorgehoben seien die Verbindungen

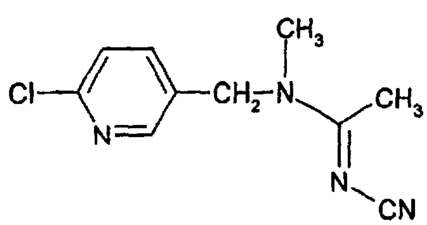

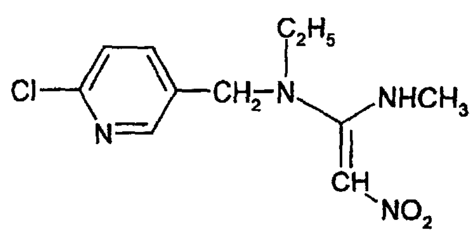

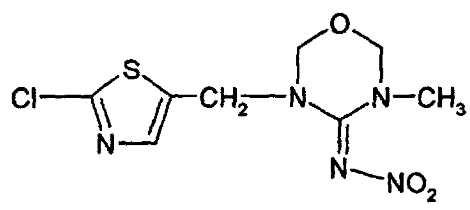

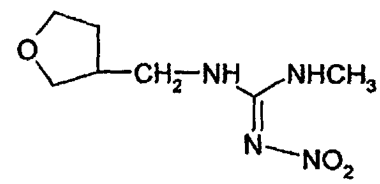

Weiterhin besonders hervorgehoben seien die Verbindungen

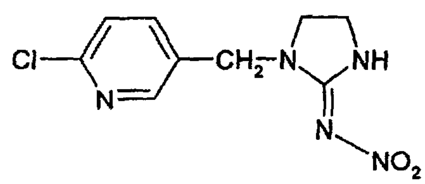

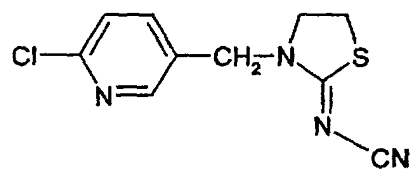

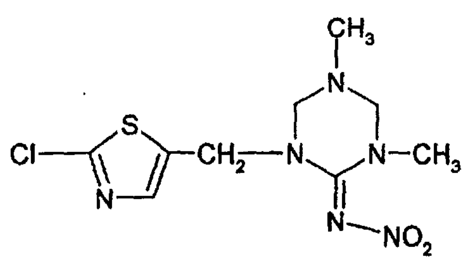

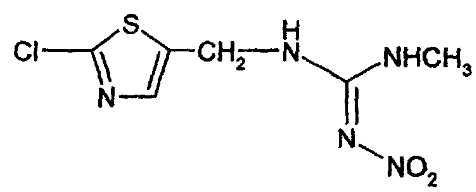

Die Wirkstoffe liegen in Konzentrationen von 0,0001 bis 10 Gew.-%, bevorzugt 0,1 bis 7,5 Gew.-%, besonders bevorzugt 0,2 bis 2 Gew.-% vor.

Wasser liegt in Konzentrationen von 20 bis 50 Gew.-%, bevorzugt von 25 bis 45 Gew.-% vor.

Als acyclische Alkohole seien genannt aliphatische $C_{1-4}$-Alkanole oder Diole wie Ethanol, Isopropanol, Diethylenglykol, ferner 2-Octyl-1-dodecanol und Tetrahydrofurfurylalkohol. Besonders genannt sei Isopropanol. Die Alkohole liegen in Konzentrationen von 20 bis 50 Gew.-%, bevorzugt 25 bis 45 Gew.-% vor. Bevorzugt wird ein 60:40 bis 50:50 Alkohol/Wasser-Gemisch eingesetzt.

Als cyclische Carbonate seien genannt Ethylencarbonat, Propylencarbonat. Besonders genannt sei Propylencarbonat. Sie liegen in Konzentrationen von 2,5 bis 30 Gew.-%, bevorzugt von 2,5 bis 20 Gew.-%, besonders bevorzugt von 5 bis 12,5 Gew.-% vor.

Als weitere Hilfsmittel kommen in Frage: Konservierungsmittel wie Benzylalkohol und Geruchs- bzw. Duftstoffe.

Sie liegen vor in einer Konzentration von 0 bis 15 Gew.-%, bevorzugt 2,5 bis 12,5 Gew.-%, besonders bevorzugt 2,5 bis 10,0 Gew.-%.

Die Summe von Wirkstoffen, Lösungsmitteln und Hilfsmitteln muß 100 Gew.-% betragen.

Weitere Hilfsmittel sind:

Verdickungsmittel sind z.B. anorganische Verdickungsmittel wie Bentonite, kolloidale Kieselsäure, Aluminiummonostearat, organische Verdickungsmittel wie Cellulosederivate, Polyvinylalkohole, Polyvinylpyrrolidone und deren Copolymere, Acrylate und Methacrylate.

Als Farbstoffe seien genannt alle zur Herstellung von Arzneimitteln zugelassenen Farbstoffe, die gelöst oder suspendiert sein können.

Als Hilfsstoffe seien genannt spreitende Öle wie Adipinsäure-di-2-ethylhexylester, Isopropylmyristat, Dipropylenglykolpelargonat, cyclische und acyclische Silikonöle wie Dimetikone, deren Derivate mit den kationischen und anionischen funktionellen Gruppen. Die genaue Beschreibung der Silikone mit kationischen Gruppen kann beispielsweise aus S. Marchioretto, J. Bakely, SOFW-Journal, 123, S. 881 (1997); B. Ziolkowsky, SOFW-Journal, 123, S. 822 (1997) und DE-PS 44 43 062 entnommen werden. Ferner deren Co- und Terpolymerisate mit Ethylenoxid, Propylenoxid und Formalin, Fettsäureester, Triglyceride, Fettalkohole.

Antioxidantien sind z.B. Sulfite oder Metabisulfite wie Kaliummetabisulfit, Ascorbinsäure, Butylhydroxytoluol, Butylhydroxyanisol, Tocopherol.

Lichtschutzmittel sind z.B. Stoffe aus der Klasse der Benzophenone oder Novantisolsäure.

Haftmittel sind z.B. polymere Verdickungsmittel, z.B. Cellulosederivate, Stärkederivate, Polyacrylate, natürliche Polymere wie Alginate, Gelatine.

Hilfsstoffe sind auch Emulgatoren wie nichtionogene Tenside, z.B. polyoxyethyliertes Rizinusöl, polyoxyethyliertes Sorbitan-monooleat, Sorbitanmonostearat, Glycerinmonostearat, Polyoxyethylstearat, Alkylphenolpolyglykolether;
ampholytische Tenside wie Di-Na-N-lauryl-β-iminodipropionat oder Lecithin;
anionaktive Tenside, wie Na-Laurylsulfat, Fettalkoholethersulfate, Mono/Dialkylpolyglykoletherorthophosphor-säureester-monoethanolaminsalz;
kationaktive Tenside wie Cetyltrimethylammoniumchlorid.

Weitere Hilfsstoffe sind Mittel mit denen die erfindungsgemäßen Formulierungen auf die Haut gesprüht oder gespritzt oder gerieben werden können. Dabei handelt es sich um die für Spraydosen benötigten üblichen Treibgase wie Propan, Butan, Dimethylether, $CO_2$ oder halogenierte Niedrigalkane, bzw. deren Mischungen untereinander.

Die Menge der genannten Hilfsmittel kann im Bereich von 0 bis 10 Gew.-%, vorzugsweise jedoch 0,025 bis 2,5 Gew.-% variiert werden.

Die erfindungsgemäßen Formulierungen eignen sich bei günstiger Warmblütertoxizität zur Bekämpfung von parasitierenden Insekten, die am Menschen vorkommen. Sie sind dabei gegen alle oder einzelne Entwicklungsstadien der Schädlinge sowie gegen resistente und normal sensible Arten der Schädlinge wirksam.

Zu den Schädlingen gehören:
Aus der Ordnung der Anoplura z.B. Pediculus spp., Pthirus spp.;
Aus der Ordnung der Siphonaptera z.B. Ctenocephalides spp., Echidnophaga spp., Ceratophyllus spp..

Besonders hervorgehoben sei die Wirkung gegen Anoplura und Siphonaptera. In diesem Zusammenhang sei die Wirkung gegen Pediculus humanus capitis (Kopflaus), Pediculus humanus corporis (Kleiderlaus) und Phthirus pubis (Filzlaus) erwähnt.

Die erfindungsgemäßen Formulierungen können zusätzlich Juvenilhormone oder juvenilhormonartige Substanzen wie z.B. Diarylether, Benzoylharnstoffe oder Triazine enthalten. Hierzu gehören insbesondere Verbindungen der folgenden Formeln:

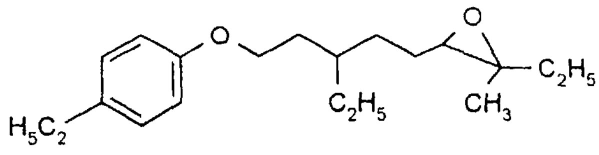

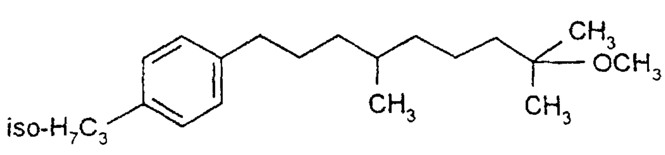

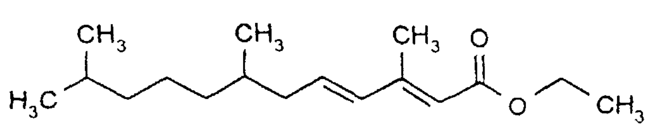

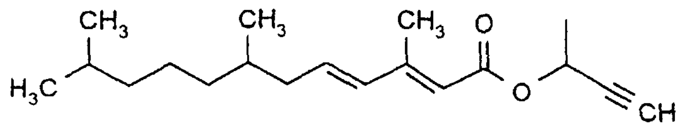

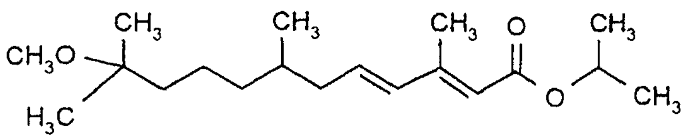

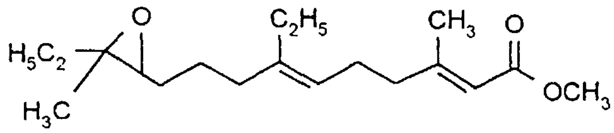

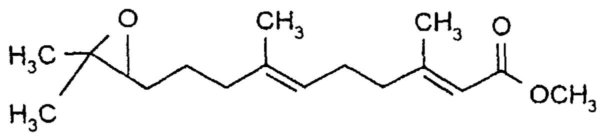

Zu den substituierten Diarylethern gehören insbesondere

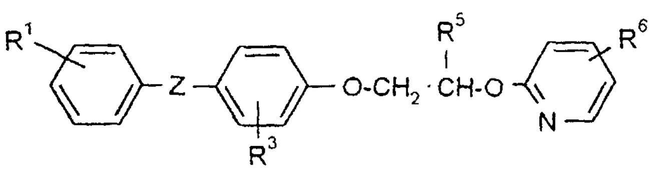

| $R^1$ | $R^3$ | $R^5$ | $R^6$ | Z |
|---|---|---|---|---|
| H | H | $CH_3$ | H | O |
| H | H | $CH_3$ | 2-Cl | O |
| 5-F | H | $CH_3$ | H | O |
| H | H | $CF_3$ | H | O |
| H | H | $C_2H_5$ | H | O |
| H | H | H | H | O |
| H | H | $CH_3$ | H | $CH_2$ |
| H | H | $CH_3$ | H | $C(CH_3)_2$ |

Zu den Benzoylharnstoffen gehören Verbindungen der Formel

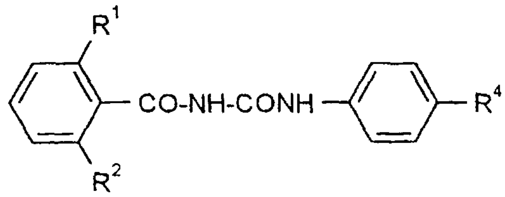

| $R^1$ | $R^2$ | $R^4$ |
|---|---|---|
| H | Cl | $CF_3$ |
| Cl | Cl | $CF_3$ |
| F | F | $CF_3$ |
| H | F | $CF_3$ |
| H | Cl | $SCF_3$ |
| F | F | $SCF_3$ |
| H | F | $SCF_3$ |
| H | Cl | $OCF_3$ |
| F | F | $OCF_3$ |
| H | F | $OCF_3$ |
| F | F | 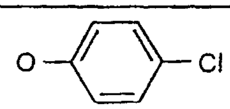 |
| F | F | 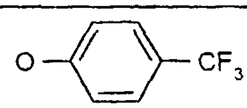 |
| F | F | 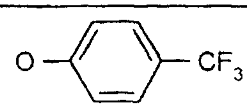 |

Zu den Triazinen gehören Verbindungen der Formel

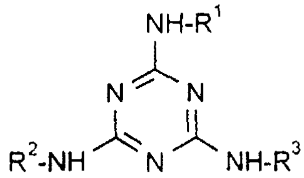

| $R^1$ | $R^2$ | $R^3$ |
|---|---|---|
| Cyclopropyl | H | H |
| Cyclopropyl | H | $CH_3$ |
| Cyclopropyl | H | $C_2H_5$ |
| Cyclopropyl | H | $C_3H_7$-n |
| Cyclopropyl | H | $C_4H_9$-n |
| Cyclopropyl | H | $C_5H_{11}$-n |
| Cyclopropyl | H | $C_6H_{13}$-n |
| Cyclopropyl | H | $C_7H_{15}$-n |
| Cyclopropyl | H | $C_8H_{17}$-n |
| Cyclopropyl | H | $C_{12}$-$H_{25}$-n |
| Cyclopropyl | H | $CH_2$-$C_4H_9$-n |
| Cyclopropyl | H | $CH_2CH(CH_3)C_2H_5$ |
| Cyclopropyl | H | $CH_2CH=CH_2$ |
| Cyclopropyl | Cl | $C_2H_5$ |
| Cyclopropyl | Cl | $C_6H_{13}$-n |
| Cyclopropyl | Cl | $C_8H_{17}$-n |
| Cyclopropyl | Cl | $C_{12}H_{25}$-n |
| Cyclopropyl | H | Cyclopropyl |
| Cyclopropyl | H | $COCH_3$ |
| Cyclopropyl | H | $COCH_3 \cdot HCl$ |
| Cyclopropyl | H | $COC_2H_5 \cdot HCl$ |
| Cyclopropyl | H | $COC_2H_5$ |
| Cyclopropyl | H | $COC_3H_7$-n |
| Cyclopropyl | H | $COC_3H_7$-i |
| Cyclopropyl | H | $COC_4H_9$-t · HCl |
| Cyclopropyl | H | $COC_4H_9$-n |
| Cyclopropyl | H | $COC_6H_{13}$-n |
| Cyclopropyl | H | $COC_{11}$-$H_{23}$-n |
| Cyclopropyl | $COCH_3$ | $COC_2H_5$ |
| Cyclopropyl | $COC_3H_7$-n | $COC_6H_{13}$-n |
| Cyclopropyl | $COCH_3$ | $COC_3H_7$-n |
| Cyclopropyl | $COC_2H_5$ | $COC_3H_7$-n |

(fortgesetzt)

| $R^1$ | $R^2$ | $R^3$ |
|---|---|---|
| Cyclopropyl | H | COCyclopropyl |
| Cyclopropyl | COCyclopropyl | COCyclopropyl |
| Cyclopropyl | $COCH_3$ | $COCH_3$ |
| Isopropyl | H | H |
| Isopropyl | H | $COCH_3$ |
| Isopropyl | H | $COC_3H_7$-n |
| Cyclopropyl | H | $CONHCH_3$ |
| Cyclopropyl | H | $CONHC_3H_7$-i |
| Cyclopropyl | $CONHCH_3$ | $CONHCH_3$ |
| Cyclopropyl | H | $SCNHCH_3$ |
| Cyclopropyl | H | $CONHCH_2CH=CH_2$ |
| Cyclopropyl | $CONHCH_2CH=CH_2$ | $CONHCH_2CH=CH_2$ |
| Cyclopropyl | $CSNHCH_3$ | $CSNHCH_3$ |

Die Menge der zusätzlichen Wirkstoffe kann 0 bis 10 Gew.-%, bezogen auf die gesamte Formulierungsmasse, vorzugsweise bis zu 7,5 %, besonders bevorzugt bis zu 5,0 % betragen.

Als erfindungsgemäß verwendbare Wirkstoffe seien genannt Imidacloprid, AKD 1022 und Ti 435.

AKD 1022 ist ein Chlornicotinylderivat mit der Formel

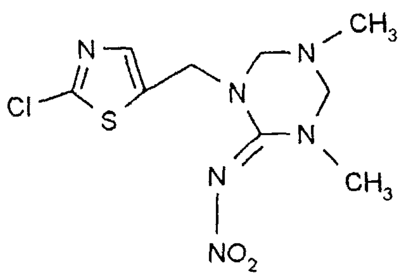

Ti 435 ist ein Chlornicotinylderivat mit der Formel

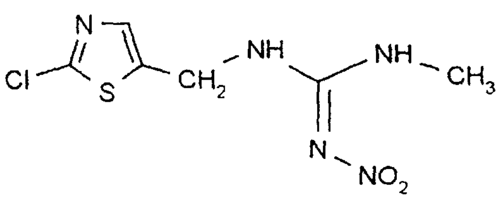

Überraschend ist, daß durch die Verwendung von Wasser gegebenenfalls in Kombination mit Spreit- und Hilfsmitteln die parastizide Wirkung erheblich verbessert wird. Dies führt zu einer Reduzierung der Wirkstoffdosierung.

In den folgenden Beispielen wird als Wirkstoff 1-[(6-Chlor-3-pyridinyl)methyl]-4,5-dihydro-N-nitro-1H-imidazol-2-amin (common name Imidacloprid), eingesetzt.

**Beispiel 1**

| Imidacloprid Lotion 1.0 % m/V | | | |
|---|---|---|---|
| Zmidacloprid | 1.000 g | | a.i. |
| Isopropyl alcohol/water (60:40) | 79.796 g | | solvent |
| Propylene carbonate | 10.000 g | | solvent |
| | 90.796 g | = 100.0 ml | |

**Beispiel 2**

| Imidacloprid Lotion 1.0 % m/V | | | |
|---|---|---|---|
| Imidacloprid | 1.000 g | | a.i. |
| Isopropyl alcohol/water (60:40) | 79.026 g | | solvent |
| Propylene carbonate | 10.000 g | | solvent |
| ®Cetiol HE*) | 1.000 g | | surfactant |
| | 91.026 g | = 100.0 ml | |

*) (Ein Polyethylenglykolmonococonant der Fa. Henkel AG)

**Beispiel 3**

| Imidacloprid Lotion 1.0 % m/V | | | |
|---|---|---|---|
| Imidacloprid | 1.000 g | | a.i. |
| Isopropyl alcohol/water (60:40) | 79.026 g | | solvent |
| Propylene carbonate | 10.000 g | | solvent |
| ®Abil Quart 3272**) | 1.000 g | | surfactant, hair conditioner |
| | 91.026 g | = 100.0 ml | |

**) (Polysiloxancopolymerisat mit quarternären Ammoniumgruppen der Fa. Goldschmidt AG).

**Beispiel 4**

| Imidacloprid Lotion 1.0 % m/V | | | |
|---|---|---|---|
| Imidacloprid | 1.000 g | | a.i. |
| Isopropyl alcohol/water (60:40) | 84.044 g | | solvent |
| Propylene carbonate | 10.000 g | | solvent |
| | 95.044 g | = 100.0 ml | |

**Beispiel 5**

| Imidacloprid Lotion 1.0 % m/V | | | |
|---|---|---|---|
| Imidacloprid | 1.000 g | | a.i. |
| Isopropyl alcohol/water (60:40) | 83.402 g | | solvent |
| Propylene carbonate | 10.000 g | | solvent |

(fortgesetzt)

| Imidacloprid Lotion 1.0 % m/V | | | |
|---|---|---|---|
| ®Cetiol HE*) | 1.000 g | | surfactant |
| | 95.402 g | = 100.0 ml | |

*) (Ein Polyethylenglykolmonococonant der Fa. Henkel AG)

**Beispiel 6**

| Imidacloprid Lotion 1.0 % m/V | | | |
|---|---|---|---|
| Imidacloprid | 1.000 g | | a.i. |
| Isopropyl alcohol/water (60:40) | 83.402 g | | solvent |
| Propylene carbonate | 10.000 g | | solvent |
| ®Abil Quart 3272**) | 1.000 g | | surfactant, hair conditioner |
| | 95.402 g | = 100.0 ml | |

**) (Polysiloxancopolymerisat mit quarternären Ammoniumgruppen der Fa. Goldschmidt AG).

**Anwendungsbeispiel A**

Pediculus / Wildpopulation

Es wurden Läuse aus einer Wildpopulation (Pediculus humanus capitis) verwendet, die den Trägern ausgekämmt und unmittelbar nach Gewinnung in die Versuche eingesetzt wurden. Die verwendeten Läuseeier (Nissen) wurden ebenfalls frisch aus dem Haar befallener Probanden entnommen und für die Prüfung bereitgestellt (eine Nisse je geschnittenem Haar).

Die Applikation erfolgte entsprechend einer praxisnahen Imitation einer Lotionsanwendung durch Tauchbehandlung von Läusen und Nissen. Adulte Läuse wurden in Testgruppen à 15 für 5 bis 10 min. den Testlösungen ausgesetzt. Die Nissen mit dem zugehörigen Trägerhaar wurden in Testgruppen von 10 für jeweils 10 min. in die Testlösungen getaucht. Nach Ablauf der jeweiligen Inkubationsphase wurden Läuse und Nissen mit Wasser gewaschen und getrocknet. Die Wirksamkeit der Behandlung wurde bei Adulten nach 3 h mit 100 % und bei den Nissen nach 12 d (Schlupfhemmung) mit 100 % bestimmt.

**Anwendungsbeispiel B**

Pediculus / Laborstamm

Es wurden Läuse aus einer Laborpopulation (Pediculus humanus humanus) verwendet, die frisch aus dem Kultivationszyklus entnommen wurden. Die verwendeten Läuseeier (Nissen) wurden ebenfalls frisch nach der Ablage durch adulte Weibchen auf Faservliesplättchen gewonnen und für die Prüfung bereitgestellt.

Die Applikation erfolgte entsprechend einer praxisnahem Imitation einer Lotionsanwendung durch Tauchbehandlung von Läusen und Nissen. Adulte Läuse wurden in Testgruppen à 20 für 10 sec. den Testlösungen ausgesetzt. Die Nissen wurden in Testgruppen von 20 für jeweils 10 min. in die Testlösungen getaucht. Nach Ablauf der jeweiligen Inkubationsphase wurden Läuse und Nissen mit Wasser gewaschen und getrocknet. Die Wirksamkeit der Behandlung wurde bei Adulten nach 24 h mit 100 % und bei den Nissen nach 12 d mit 100 % (Schlupfhemmung) bestimmt.

**Patentansprüche**

1. Wasserhaltige Formulierungen zur dermalen Anwendung zur Bekämpfung parasitierender Insekten und Milben an Menschen mittels Agonisten oder Antagonisten der nicotinergen Acetylcholinrezeptoren, der folgenden Zusammensetzung

a - Agonisten oder Antagonisten der nicotinergen Acetylcholinrezeptoren von Insekten in einer Konzentration von 0,0001 bis 10 Gew.-%, bezogen auf das Gesamtgewicht der Formulierung;

b - Wasser in einer Konzentration von 20 bis 50 Gew.-%, bezogen auf das Gesamtgewicht der Formulierung;

c - acyclische Alkohole in einer Konzentration von 20 bis 50 Gew.-%, bezogen auf das Gesamtgewicht der Formulierung;

d - cyclische Carbonate in einer Konzentration von 2,5 bis zu 20,0 Gew.-%, bezogen auf das Gesamtgewicht der Formulierung;

e - gegebenenfalls weitere Hilfsmittel aus der Gruppe Verdickungsmittel, Spreitmittel, Farbstoffe, Antioxidantien, Treibmittel, Konservierungsstoffe, Geruchsstoffe, Duftstoffe, Lichtschutzmittel, Haftmittel, Emulgatoren, in einer Konzentration von 0 bis zu 30 Gew.-%, bezogen auf das Gesamtgewicht der Formulierung.

**2.** Wasserhaltige Formulierungen gemäß Anspruch 1, in welchen als Wirkstoff a Verbindungen der allgemeinen Formel (I) eingesetzt werden:

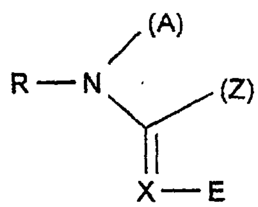

(I),

in welcher

R für Wasserstoff, gegebenenfalls substituierte Reste der Gruppe Acyl, Alkyl, Aryl, Aralkyl, Heteroaryl oder Heteroarylalkyl steht;

A für eine monofunktionelle Gruppe aus der Reihe Wasserstoff, Acyl, Alkyl, Aryl steht oder für eine bifunktionelle Gruppe steht, die mit dem Rest Z verknüpft ist;

E für einen elektronenziehenden Rest steht;

X für die Reste -CH= oder =N- steht, wobei der Rest -CH= anstelle eines H-Atoms mit dem Rest Z verknüpft sein kann;

Z für eine monofunktionelle Gruppe aus der Reihe Alkyl, -O-R, -S-R,

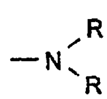

steht
wobei die Reste R gleich oder verschieden sind und die oben angegebene Bedeutung haben;
oder für eine bifunktionelle Gruppe steht, die mit dem Rest A oder dem Rest X verknüpft ist.

**3.** Formulierungen gemäß Anspruch 2, in welchen als Wirkstoff a eine oder mehrere Verbindungen der Formel (I), in welcher die Reste folgende Bedeutung haben, eingesetzt werden:

R steht für Wasserstoff sowie für gegebenenfalls substituierte Reste aus der Reihe Acyl, Alkyl, Aryl, Aralkyl, Heteroaryl, Heteroarylalkyl, Heterocyclylalkyl, wobei
Acylreste Formyl, Alkylcarbonyl, Arylcarbonyl, Alkylsulfonyl, Arylsulfonyl, (Alkyl-)-(Aryl-)-phosphoryl

sind, die ihrerseits substituiert sein können,
Alkyl $C_{1-10}$-Alkyl sind, die ihrerseits substituiert sein können,
Aryl Phenyl, Naphthyl sind,
Aralkyl Phenylmethyl, Phenylethyl sind,
Heteroaryl Thienyl, Furyl, Thiazolyl, Imidazolyl, Pyridyl, Benzthiazolyl sind,
Heteroarylalkyl Heteroaryhnethyl, Heteroarylethyl sind, wobei die Heteroarylreste bis zu 6 Ringatome und N, O oder S als Heteroatome enthalten,
Heterocyclylalkyl Tetrahydrofuranylmethyl ist,
Substituenten sind:

Alkyl mit 1 bis 4 Kohlenstoffatomen, Alkoxy mit 1 bis 4 Kohlenstoffatomen, Alkylthio mit 1 bis 4 Kohlenstoffatomen, Halogenalkyl mit 1 bis 4 Kohlenstoffatomen und 1 bis 5 Halogenatomen, wobei die Halogenatome gleich oder verschieden sind und als Halogenatome Fluor, Chlor oder Brom stehen, Cyano; Nitro; Amino; Monoalkyl- und Dialkylamino mit 1 bis 4 Kohlenstoffatomen je Alkylgruppe, Carboxyl; Carbalkoxy mit 2 bis 4 Kohlenstoffatomen; Sulfo ($-SO_3H$); Alkylsulfonyl mit 1 bis 4 Kohlenstoffatomen, Phenylsulfonyl, Chlorpyridylamino und Chlorpyridylmethylamino.

A steht für Wasserstoff sowie für gegebenenfalls substituierte Reste aus der Reihe Acyl, Alkyl, Aryl, die die bei R angegebenen Bedeutungen haben. A steht ferner für eine bifunktionelle Gruppe. Genannt sei gegebenenfalls substituiertes Alkylen mit 1-4, insbesondere 1-2 C-Atomen, wobei als Substituenten die weiter oben aufgezählten Substituenten genannt seien und wobei die Alkylengruppen durch Heteroatome aus der Reihe N, O oder S unterbrochen sein können.

A und Z können gemeinsam mit den Atomen, an welche sie gebunden sind, einen gesättigten oder ungesättigten heterocyclischen Ring bilden. Der heterocyclische Ring kann weitere 1 oder 2 gleiche oder verschiedene Heteroatome und/oder Heterogruppen enthalten. Als Heteroatome stehen vorzugsweise Sauerstoff, Schwefel oder Stickstoff und als Heterogruppen N-Alkyl, wobei Alkyl der N-Alkyl-Gruppe 1 bis 4 Kohlenstoffatome enthält.
Als heterocyclische Ringe seien genannt: Pyrrolidin, Piperidin, Piperazin, Hexamethylenimin, Hexahydro-1,3,5-triazin, Morpholin, Oxadiazin, die gegebenenfalls durch Methyl substituiert sein können.

E steht für einen elektronenziehenden Rest, vorzugsweise $NO_2$, CN, Halogenalkylcarbonyl wie 1-5-Halogen-$C_{1-4}$-carbonyl, insbesondere $COCF_3$, sowie Alkylsuslfonyl und Halogenalkylsulfonyl wie 1-5-Halogen-$C_1$-$C_4$-sulfonyl, insbesondere $SO_2CF_3$.

X steht für -CH= oder -N= .

Z steht für gegebenenfalls substituierte Reste Alkyl, -OR, -SR, -NRR (die Reste R sind gleich oder verschieden), wobei R und die Substituenten bevorzugt die oben angegebene Bedeutung haben.

Z kann außer dem obengenannten Ring gemeinsam mit dem Atom, an welches es gebunden ist und dem Rest

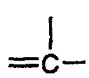

an der Stelle von X einen gesättigten oder ungesättigten heterocyclischen Ring bilden. Der heterocyclische Ring kann weitere 1 oder 2 gleiche oder verschiedene Heteroatome und/oder Heterogruppen enthalten. Als Heteroatome stehen Sauerstoff, Schwefel oder Stickstoff und als Heterogruppen N-Alkyl, wobei die Alkyl oder N-Alkyl-Gruppe 1 bis 4 Kohlenstoffatome enthält.
Als heterocyclische Ringe seien Pyrrolidin, Piperidin, Piperazin, Hexamethylenimin, Morpholin und N-Methylpiperazin genannt.

**4.** Formulierungen gemäß Anspruch 2, in welchen als Wirkstoff a eine oder mehrere Verbindungen der Formel (I), in welcher die Reste folgende Bedeutung haben, enthalten sind:

R steht für gegebenenfalls substituierte Reste aus der Reihe Heteroarylmethyl oder Heteroarylethyl, wobei als Heteroaryl genannt sei:

Tienyl, Furyl, Thiazolyl, Imidazolyl, Pyridyl, Benzthiazolyl, wobei

Substituenten sind:

Methyl, Ethyl, Methoxy, Ethoxy, Methylthio, Ethylthio, Trifluormethyl; Hydroxy; Fluor, Chlor und Brom; Cyano; Nitro; Amino;

A steht für Wasserstoff sowie für eine mit dem Rest Z verknüpfte bifunktionelle, gegebenenfalls substituierte Alkylengruppe mit 2 C-Atomen, wobei als Substituenten die weiter oben aufgezählten Substituenten genannt seien und wobei die Alkylengruppe durch 1 Heteroatom aus der Reihe N, O oder S unterbrochen sein kann,

A und Z können gemeinsam mit den Atomen, an welche sie gebunden sind, einen gesättigten oder ungesättigten 5- oder 6-gliedrigen heterocyclischen Ring bilden. Der heterocyclische Ring kann weitere 1 oder 2 gleiche oder verschiedene Heteroatome und/oder Heterogruppen enthalten. Als Heteroatome stehen Sauerstoff, Schwefel oder Stickstoff und als Heterogruppen N-Alkyl, wobei Alkyl der N-Alkyl-Gruppe 1 oder 2 Kohlenstoffatome enthält.

E steht für $NO_2$, CN.

X steht für -CH= oder -N= .

Z steht für gegebenenfalls substituierte Reste Alkyl, -OR',-SR', -NR'R' (die Reste R' sind gleich oder verschieden), wobei R' und die Substituenten folgende Bedeutung haben:

R' steht für Wasserstoff sowie für gegebenenfalls substituierte Reste aus der Reihe Acyl, Alkyl, Aryl, Aralkyl, Heteroaryl, Heteroarylalkyl.
Als Acylreste seien genannt Formyl, Alkylcarbonyl, Arylcarbonyl, Alkylsulfonyl, Arylsulfonyl, (Alkyl-) -(Aryl-)-phosphoryl.
Als Alkyl sei $C_{1-4}$-Alkyl genannt.
Als Aryl sei Phenyl genannt.
Als Aralkyl seien genannt Phenylmethyl, Phenylethyl.
Als Heteroarylalkyl seien genannt Heteroarylmethyl, Heteroarylethyl, wobei als Heteroaryl genannt sei Thienyl, Furyl, Thiazolyl, Imidazolyl, Pyridyl, Benzthiazolyl.
Als Substituenten der Reste R' seien aufgeführt:

Methyl, Ethyl, Methoxy, Ethoxy, Methylthio, Ethylthio, Halogenalkyl mit 1 oder 2 Kohlenstoffatomen und 1 bis 5 Halogenatomen, wobei die Halogenatome gleich oder verschieden sind und als Halogenatome Fluor, Chlor oder Brom stehen, Hydroxy; Fluor, Chlor und Brom; Cyano, Nitro, Amino, Monoalkyl- und Dialkylamino mit vorzugsweise 1 oder 2 Kohlenstoffatomen je Alkylgruppe, Carboxyl; Carbalkoxy mit 2 oder 3 Kohlenstoffatomen, Sulfo ($-SO_3H$); Alkylsulfonyl mit 1 oder 2 Kohlenstoffatomen, Phenylsulfonyl Chlorpyridylamino und Chlorpyridylmethylamino.

**5.** Formulierungen gemäß Anspruch 1, in welchen als Wirkstoffe a eine oder mehrere der Verbindungen der allgemeinen Formeln (II), (III) und (IV) enthalten sind:

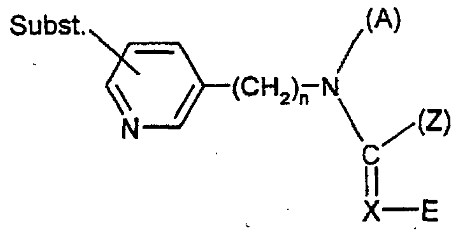

(II),

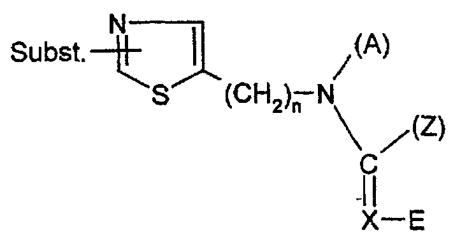

(III),

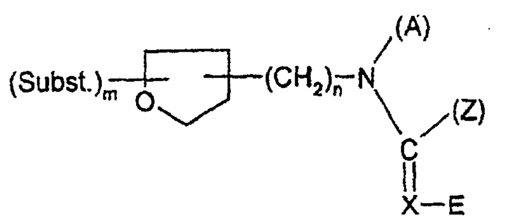

(IV),

wobei in Formeln (H), (III) und (IV)

n für 1 oder 2 steht,

m für 0, 1 oder 2 steht,

Subst. für einen der in den Ansprüchen 3 und 4 aufgeführten Substituenten steht,
A, Z, X und E die in Ansprüchen 2 bis 4 angegebenen Bedeutungen haben.

**6.** Formulierungen gemäß Anspruch 1, wobei als Wirkstoff a eine oder mehrere der folgenden Verbindungen enthalten sind:

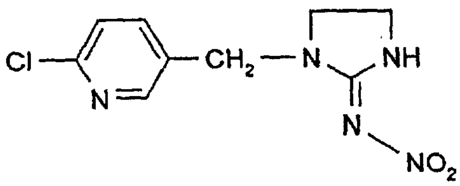

Imidacloprid

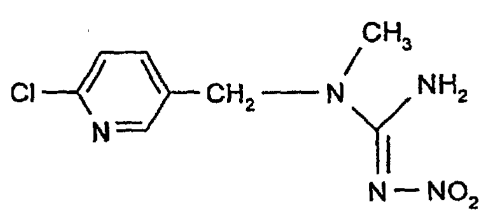

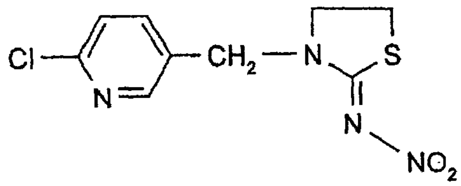

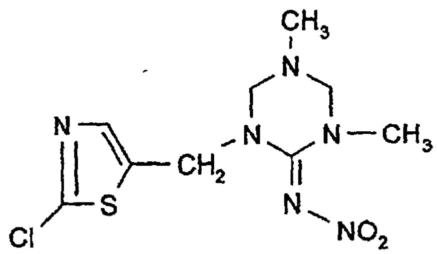

AKD 1022

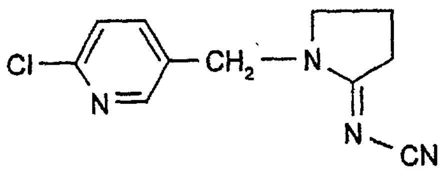

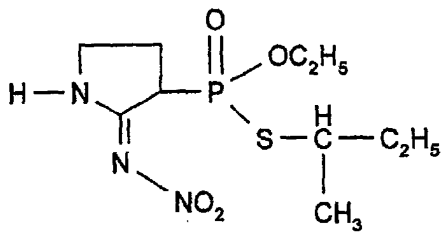

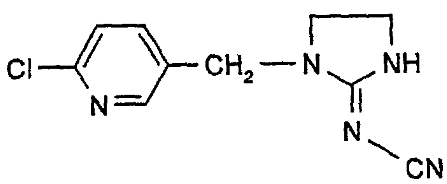

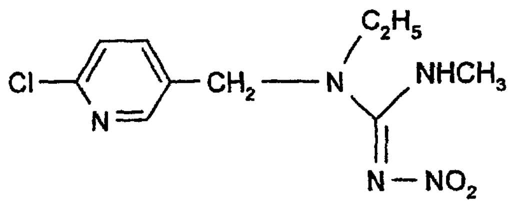

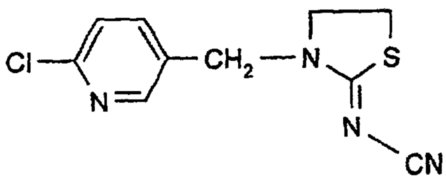

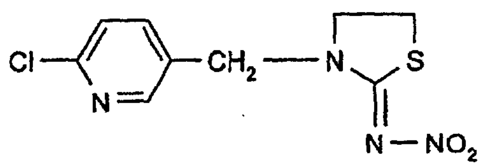

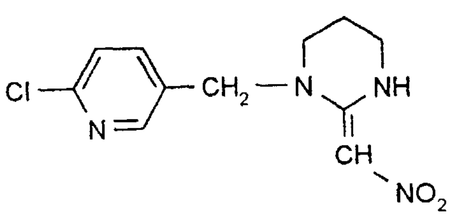

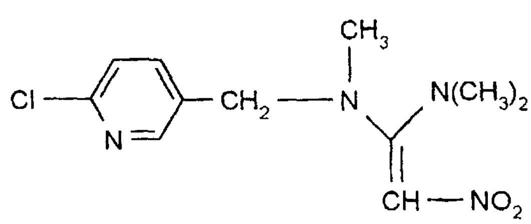

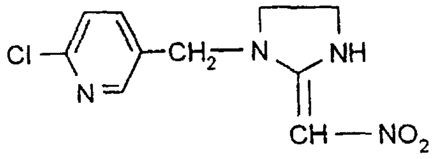

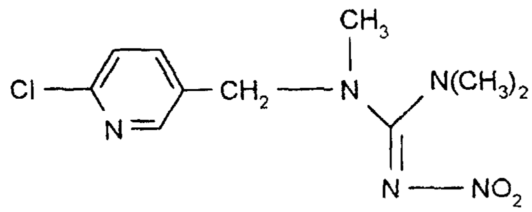

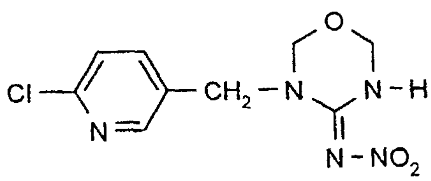

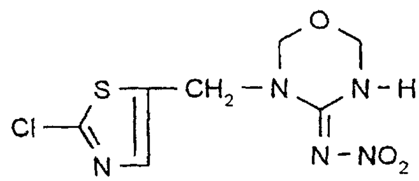

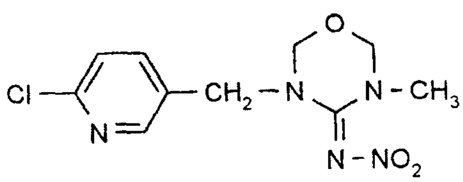

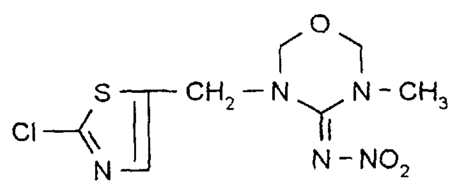

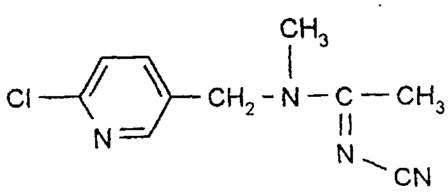

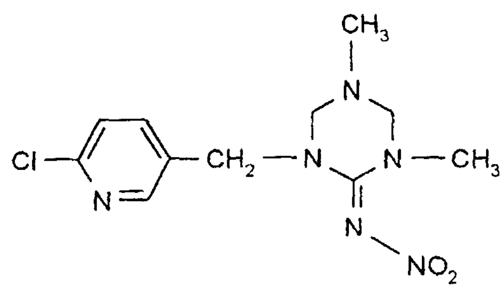

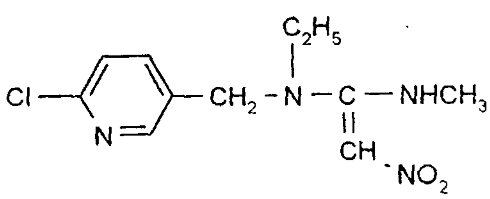

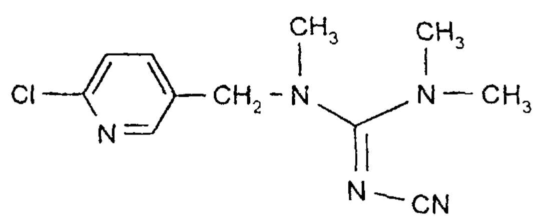

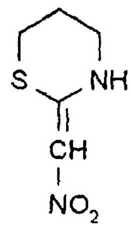

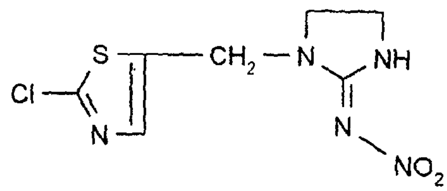

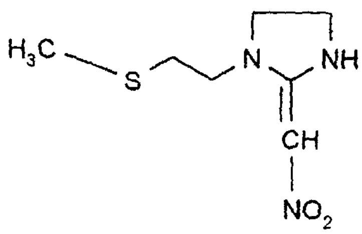

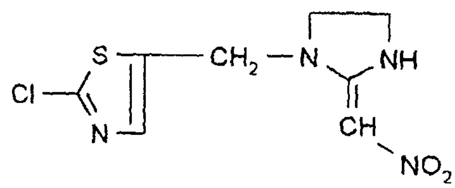

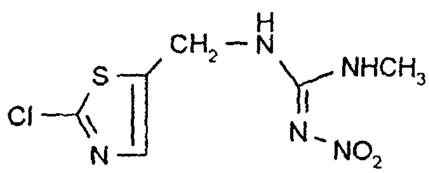

Ti435

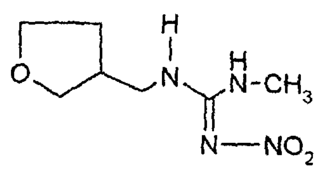

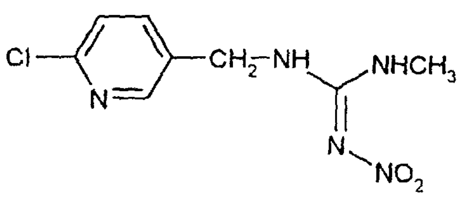

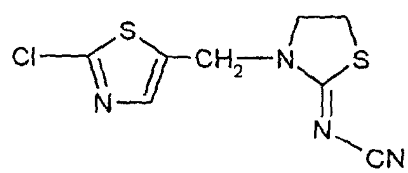

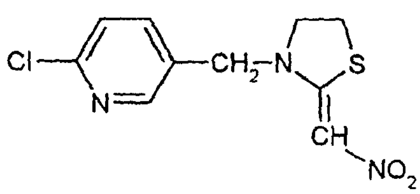

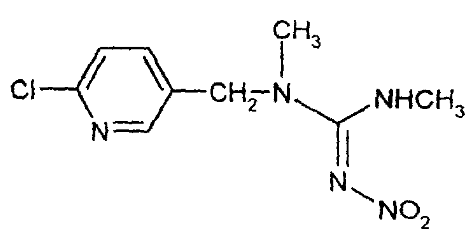

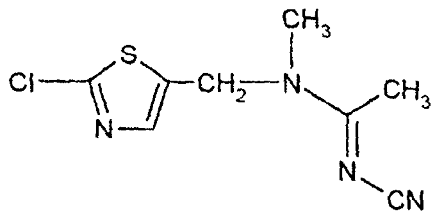

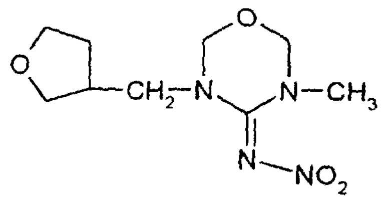

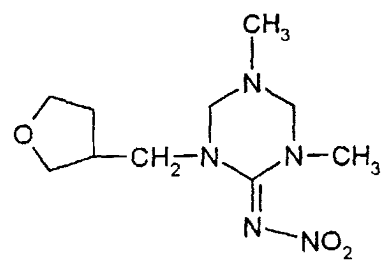

**7.** Verfahren zur Herstellung der Mittel gemäß Anspruch 1, **dadurch gekennzeichnet, daß** man den oder die Wirkstoff(e) mit Wasser und dem oder den angegebenen Lösungsmitteln zu einer homogenen Lösung vermischt und gegebenenfalls die weiteren Hilfsstoffe zusetzt.

## Claims

**1.** Water-containing formulations for dermal application for the control of parasitic insects and mites on humans by means of agonists or antagonists of the nicotinic acetylcholine receptors, of the following composition

a - agonists or antagonists of the nicotinic acetylcholine receptors of insects in a concentration of from 0.0001 to 10 % by weight, based on the overall weight of the formulation;

b - water in a concentration of from 20 to 50 % by weight, based on the overall weight of the formulation;

c - acyclic alcohols in a concentration of from 20 to 50 % by weight, based on the overall weight of the formulation;

d - cyclic carbonates in a concentration of from 2.5 up to 20.0 % by weight, based on the overall weight of the formulation;

e - optionally further auxiliaries from the group consistiing of thickeners, spreading agents, colorants, antioxidants, propellants, preservatives, perfumes, fragrances, light stabilizers, tackifiers, emulsifiers, in a concentration of from 0 up to 30 % by weight, based on the overall weight of the formulation.

**2.** Water-containing formulations according to Claim 1, in which the active compounds a used are compounds of the general formula (I):

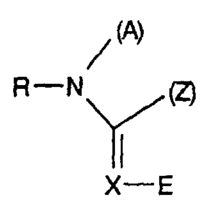

(I),

in which

R represents hydrogen, optionally substituted radicals from the group consisting of acyl, alkyl, aryl, aralkyl, heteroaryl or heteroarylalkyl;

A represents a monofunctional group from the group consisting of hydrogen, acyl, alkyl, aryl or represents a bifunctional group which is linked to the radical Z;

E represents an electron-withdrawing radical;

X represents the radicals -CH= or =N- where the radical -CH= may be linked to the radical Z instead of an H atom;

Z represents a monofunctional group from the group consisting of alkyl, -O-R, -S-R,

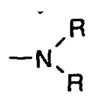

where the radicals R are identical or different and are each as defined above;
or represents a bifunctional group which is linked to the radical A or the radical X.

**3.** Formulations according to Claim 2, in which the active compound a used is one or more compounds of the formula (I) in which the radicals are as defined below:

R represents hydrogen and also represents optionally substituted radicals from the group consisting of acyl, alkyl, aryl, aralkyl, heteroaryl, heteroarylalkyl, heterocyclylalkyl, acyl radicals being formyl, alkylcarbonyl, arylcarbonyl, alkylsulphonyl, arylsulphonyl, (alkyl-)-(aryl-)-phosphoryl, which for their part may be substituted,
alkyls being $C_{1-10}$-alkyls which for their part may be substituted,
aryls being phenyl, naphthyl,
aralkyls being phenylmethyl, phenylethyl,
heteroaryls being thienyl, furyl, thiazolyl, imidazolyl, pyridyl, benzothiazolyl,
heteroarylalkyls being heteroarylmethyl, heteroarylethyl, where the heteroaryl radicals contain up to 6 ring atoms and N, O or S as heteroatoms,
heterocyclylalkyl being tetrahydrofuranylmethyl,
substituents being:

alkyl having 1 to 4 carbon atoms, alkoxy having 1 to 4 carbon atoms, alkylthio having 1 to 4 carbon atoms, halogenoalkyl having 1 to 4 carbon atoms and 1 to 5 halogen atoms, where the halogen atoms are identical or different and are fluorine, chlorine or bromine, cyano; nitro; amino; monoalkyl- and dialkylamino having 1 to 4 carbon atoms per alkyl group, carboxyl; carbalkoxy having 2 to 4 carbon atoms; sulpho ($-SO_3H$); alkylsulphonyl having 1 to 4 carbon atoms, phenylsulphonyl, chloropyridylamino and chloropyridylmethylamino.

A represents hydrogen and also represents optionally substituted radicals from the group consisting of acyl, alkyl, aryl, which are as defined for R. A furthermore represents a bifunctional group. Mention may be made of optionally substituted alkylene having 1-4, in particular 1-2, carbon atoms, where the substituents which may be mentioned are the substituents listed further above and where the alkylene groups may be interrupted by heteroatoms from the group consisting of N, O and S.

A and Z together with the atoms to which they are attached may form a saturated or unsaturated heterocyclic ring. The heterocyclic ring may contain a further 1 or 2 identical or different heteroatoms and/or hetero groups. Preferred heteroatoms are oxygen, sulphur or nitrogen and preferred hetero groups are N-alkyl, where the alkyl of the N-alkyl group contains 1 to 4 carbon atoms.
Heterocyclic rings which may be mentioned are: pyrrolidine, piperidine, piperazine, hexamethyleneimine, hexahydro-1,3,5-triazine, morpholine, oxadiazine, each of which may optionally be substituted by methyl.

E represents an electron-withdrawing radical, preferably $NO_2$, CN, halogenoalkylcarbonyl such as 1-5-halogeno-$C_{1-4}$-carbonyl, in particular $COCF_3$, and also alkylsulphonyl and halogenoalkylsulphonyl, such as 1-5-halogeno-$C_1$-$C_4$-sulphonyl, in particular $SO_2CF_3$.

X represents -CH= or -N= .

Z represents optionally substituted radicals alkyl, -OR, -SR, -NRR (the radicals R are identical or different), where R and the substituents are preferably as defined above.

Z may, in addition to the abovementioned ring, form a saturated or unsaturated heterocyclic ring together with the atom to which it is attached and the radical

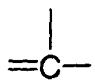

instead of X. The heterocyclic ring may contain a further 1 or 2 identical or different heteroatoms and/or hetero groups. Suitable heteroatoms are oxygen, sulphur or nitrogen and suitable hetero groups are N-alkyl, where the alkyl or N-alkyl group contains 1 to 4 carbon atoms. Heterocyclic rings which may be mentioned are pyrrolidine, piperidine, piperazine, hexamethyleneimine, morpholine and N-methylpiperazine.

**4.** Formulations according to Claim 2, which contain as active compound a one or more compounds of the formula (I) in which the radicals are as defined below:

R represents optionally substituted radicals from the group consisting of heteroarylmethyl and heteroarylethyl, where heteroaryls which may be mentioned are:

thienyl, furyl, thiazolyl, imidazolyl, pyridyl, benzothiazolyl,

substituents being:

methyl, ethyl, methoxy, ethoxy, methylthio, ethylthio, trifluoromethyl; hydroxyl; fluorine, chlorine and bromine; cyano; nitro; amino;

A represents hydrogen and also represents a bifunctional, optionally substituted alkylene group having 2 carbon atoms which is attached to the radical Z, where the substituents which may be mentioned are the substituents listed further above and where the alkylene group may be interrupted by 1 heteroatom from the group consisting of N, O and S,

A and Z Z together with the atoms to which they are attached may form a saturated or unsaturated 5- or 6-membered heterocyclic ring. The heterocyclic ring may contain a further 1 or 2 identical or different heteroatoms and/or hetero groups. Suitable heteroatoms are oxygen, sulphur or nitrogen and suitable hetero groups are N-alkyl, where the alkyl of the N-alkyl group contains 1 or 2 carbon atoms.

E represents $NO_2$, CN.

X represents -CH= or -N= .

Z represents optionally substituted radicals alkyl, -OR', -SR', -NR'R' (the radicals R' are identical or different), where R' and the substituents are as defined below:

R' represents hydrogen and also represents optionally substituted radicals from the group consisting of acyl, alkyl, aryl, aralkyl, heteroaryl, heteroarylalkyl.
Acyl radicals which may be mentioned are formyl, alkylcarbonyl, arylcarbonyl, alkylsulphonyl, arylsulphonyl, (alkyl-)-(aryl-)-phosphoryl.
Alkyl which may be mentioned is $C_{1-4}$-alkyl.

Aryl which may be mentioned is phenyl.
Aralkyls which may be mentioned are phenylmethyl, phenylethyl.
Heteroarylalkyls which may be mentioned are heteroarylmethyl, heteroarylethyl, where the heteroaryls which may be mentioned are thienyl, furyl, thiazolyl, imidazolyl, pyridyl, benzothiazolyl.
Substituents of the radicals R' which may be mentioned are:

methyl, ethyl, methoxy, ethoxy, methylthio, ethylthio, halogenoalkyl having 1 or 2 carbon atoms and 1 to 5 halogen atoms, where the halogen atoms are identical or different and are fluorine, chlorine or bromine, hydroxyl; fluorine, chlorine and bromine; cyano, nitro, amino, monoalkyl- and dialkylamino preferably having 1 or 2 carbon atoms per alkyl group, carboxyl; carbalkoxy having 2 or 3 carbon atoms, sulpho ($-SO_3H$); alkylsulphonyl having 1 or 2 carbon atoms, phenylsulphonyl, chloropyridylamino and chloropyridylmethylamino.

**5.** Formulations according to Claim 1, which contain as active compounds a one or more of the compounds of the general formulae (II), (III) and (IV):

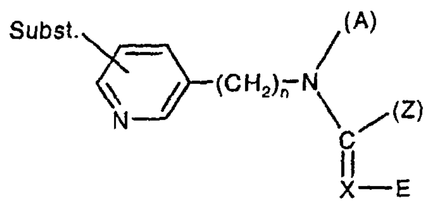

(II),

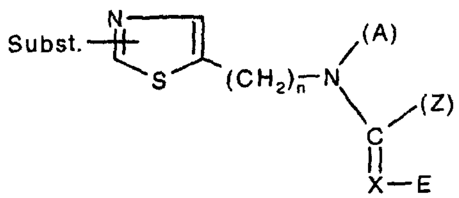

(III),

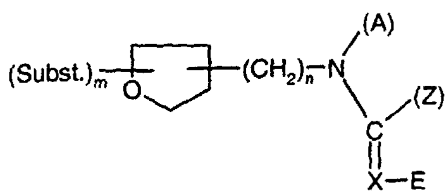

(IV),

where in the formulae (II), (III) and (IV)

n represents 1 or 2,

m represents 0, 1 or 2,

Subst. represents one of the substituents listed in Claims 3 and 4,
A, Z, X and E are each as defined in Claims 2 to 4.

**6.** Formulations according to Claim 1, containing as active compound a one or more of the following compounds:

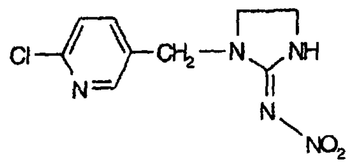

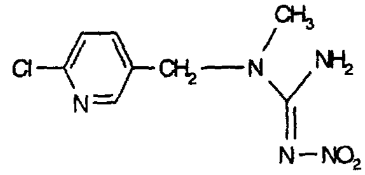

imidacloprid

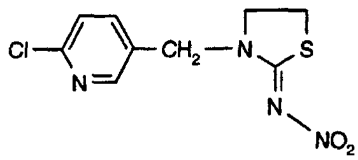

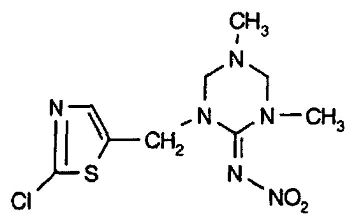

AKD 1022

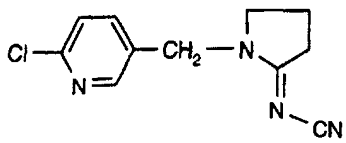

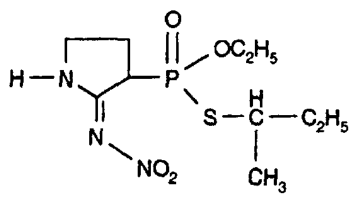

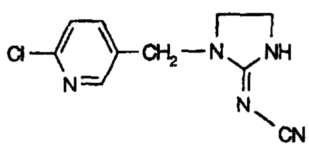

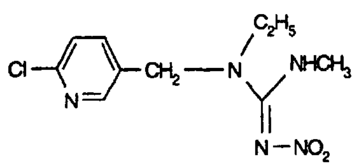

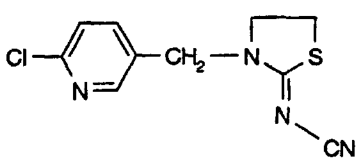

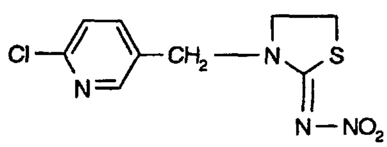

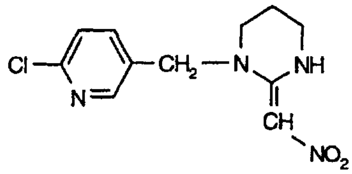

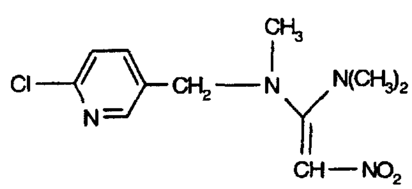

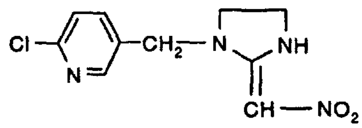

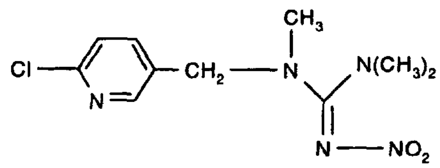

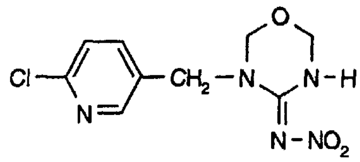

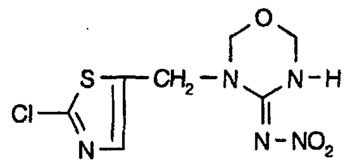

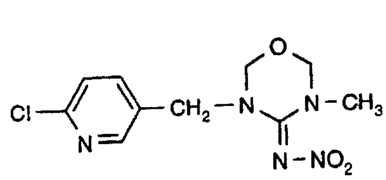

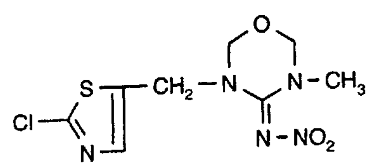

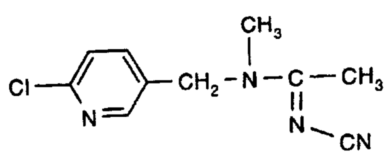

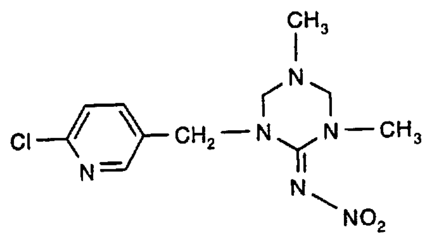

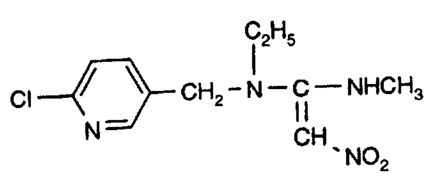

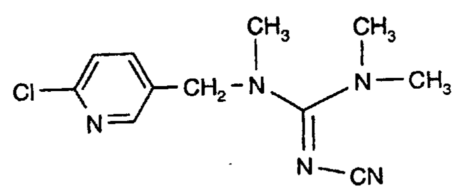

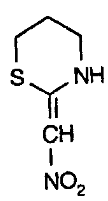

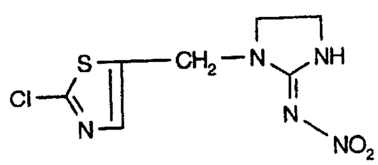

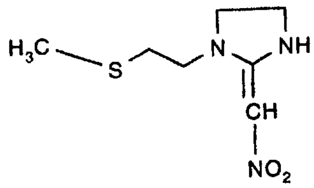

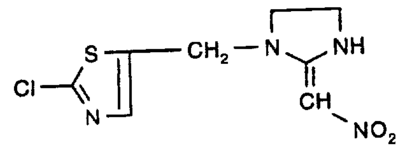

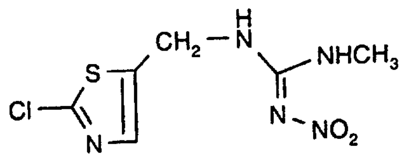

Ti435

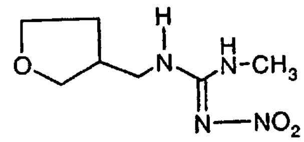

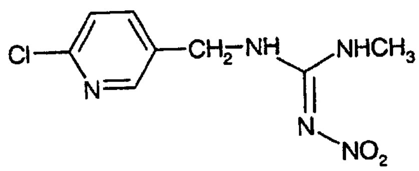

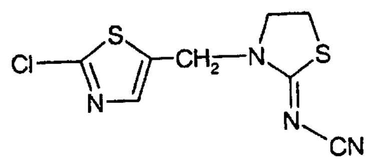

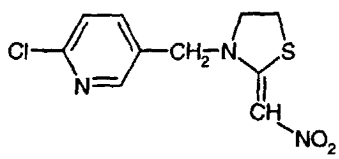

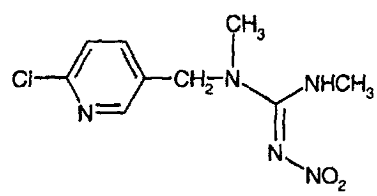

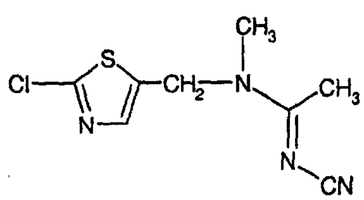

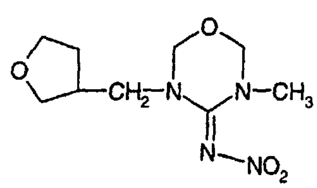

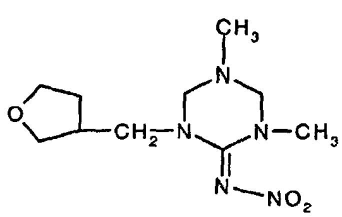

**7.** Process for preparing the compositions according to Claim 1, **characterized in that** the active compound(s) is/are admixed with water and the stated solvent(s) to give a homogeneous solution and, if appropriate, the other

auxiliaries are added.

**Revendications**

1. Formulations aqueuses pour l'utilisation dermique pour lutter contre les insectes parasites et les acariens, à l'aide d'agonistes ou d'antagonistes des récepteurs nicotiniques de l'acétylcholine, de la composition suivante :

   a- des agonistes ou antagonistes des récepteurs nicotiniques de l'acétylcholine des insectes, en une concentration allant de 0,0001 à 10% en poids, sur base du poids total de la formulation ;
   b- de l'eau en une concentration allant de 20 à 50% en poids, sur base du poids total de la formulation ;
   c- des alcools acycliques en une concentration allant de 20 à 50% en poids, sur base du poids total de la formulation ;
   d- des carbonates cycliques en une concentration allant de 2,5 à 20,0% en poids, sur base du poids total de la formulation ;
   e- le cas échéant, d'autres auxiliaires du groupe des agents épaississants, des agents d'étalement, des colorants, des antioxydants, des agents propulseurs, des agents de conservation, des substances odorantes, des parfums, des agents de protection contre la lumière, des agents d'adhérence, des émulsionnants, en une concentration allant de 0 à 30% en poids, sur base du poids total de la formulation.

2. Formulations aqueuses suivant la revendication 1, dans lesquelles comme agent actif a, on met en oeuvre des composés de la formule générale (I) :

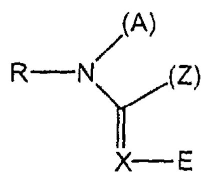

(I)

   dans laquelle :

   R représente l'atome d'hydrogène, des restes le cas échéant substitués, du groupe des radicaux acyle, alcoyle, aryle, aralcoyle, hétéroaryle ou hétéroaylalcoyle ;
   A représente un radical monofonctionnel de la série de l'atome d'hydrogène, des radicaux acyle, alcoyle, aryle ou un radical bifonctionnel, qui est relié au reste Z ;
   E représente un reste électrocapteur ;
   X représente le reste -CH= ou =N-, où le reste -CH= peut être relié au reste Z à la place de l'atome d'hydrogène ;
   Z représente un radical monofonctionnel de la série des radicaux alcoyle, -O-R, -S-R, $-NR_2$, où les restes R sont identiques ou différents et ont la signification indiquée ci-dessus, ou un radical bifonctionnel, qui est relié au reste A ou au reste X.

3. Formulations suivant la revendication 2, dans lesquelles on met en ouvre comme agent actif a, un ou plusieurs composés de la formule (I), dans laquelle les restes ont la signification suivante :

   R représente l'atome d'hydrogène ainsi que des restes le cas échéant substitués, du groupe des radicaux acyle, alcoyle, aryle, aralcoyle, hétéroaryle, hétéroaylalcoyle, hétérocyclylalcoyle, où
   les restes acyle sont les radicaux formyle, alcoylcarbonyle, arylcarbonyle, alcoylsulfonyle, arylsulfonyle, (alcoyl)(aryl)phosphoryle, qui peuvent être à leur tour, substitués ;
   les restes alcoyle sont les radicaux alcoyle en $C_{1-10}$, qui peuvent être à leur tour, substitués ;
   les restes aryle sont les radicaux phényle, naphtyle ;
   les restes aralcoyle sont les radicaux phénylméthyle, phényléthyle ;
   les restes hétéroaryle sont les radicaux thiényle, furyle, thiazolyle, imidazolyle, pyridyle, benzthiazolyle ;
   les restes hétéroarylalcoyle sont les radicaux hétéroarylméthyle, hétéroaryléthyle, où l'hétéroaryle contient jusqu'à 6 atomes cycliques et N, O ou S ;
   le reste hétérocyclylalcoyle est le radical tétrahydrofurannylméthyle ;

les substituants sont :

un radical alcoyle avec 1 à 4 atomes de carbone, un radical alcoxy avec 1 à 4 atomes de carbone, un radical alcoylthio avec 1 à 4 atomes de carbone, un radical halogénoalcoyle avec de préférence 1 à 4 atomes de carbone et 1 à 5 atomes d'halogène, où les atomes d'halogène sont identiques ou différents et comme atome d'halogène, on trouve les atomes de fluor, de chlore ou de brome, le radical cyano, nitro, le radical amino, les radicaux monoalcoyl- et dialcoylamino, avec 1 à 4 atomes de carbone par radical alcoyle, le radical carboxyle, un radical carbalcoxy, avec 2 à 4 atomes de carbone, le radical sulfo ($-SO_3H$), un radical alcoylsulfonyle avec 1 à 4 atomes de carbone, le radical phénylsulfonyle, chloropyridylamino et chloropyridylméthylamino ;

A représente l'atome d'hydrogène ainsi que un reste le cas échéant substitué de la série des radicaux acyle, alcoyle, aryle, qui a les significations données pour R. A représente de plus, un radical bifonctionnel. On citera les radicaux alcoylène substitués avec 1-4, en particulier 1-2 atomes C, où comme substituant, on indiquera les substituants énumérés ci-dessus et où les radicaux alcoylène peuvent être interrompus par des hétéroatomes de la série N, O ou S ;

A et Z peuvent former ensemble, avec les atomes sur lesquels ils sont liés, un hétérocycle saturé ou insaturé. L'hétérocycle peut contenir 1 ou 2 hétéroatomes et/ou hétérogroupes identiques ou différents. Comme hétéroatomes, on indique de préférence, l'atome d'oxygène, de soufre ou d'azote et comme hétérogroupe, N-alcoyle, où le radical alcoyle du radical N-alcoyle contient 1 à 4 atomes de carbone.

Comme hétérocycle, on citera les radicaux pyrrolidine, pipéridine, pipérazine, hexaméthylèneimine, hexahydro-1,3,5-triazine, morpholine, oxadiazine, qui peuvent être le cas échéant, substitués par le radical méthyle.

E représente un reste électrocapteur, de préférence les radicaux $NO_2$, CN, halogénoalcoylcarbonyle comme (1-5-halogéno)-$C_{1-4}$-carbonyle, en particulier $COCF_3$ ainsi que les radicaux alcoylsulfonyle et halogénoalcoylsulfonyle comme (1-5-halogéno)-$C_{1-4}$-sulfonyle, en particulier $SO_2CF_3$.

X représente le reste -CH= ou =N-.

Z représente les restes le cas échéant substitués alcoyle, -O-R, -S-R, -NRR (les restes R sont identiques ou différents), où R et les substituants ont de préférence, la signification indiquée ci-dessus.

Z peut former en plus du cycle indiqué ci-dessus, avec l'atome sur lequel il est lié et le reste =C à la place de X, un hétérocycle saturé ou insaturé. L'hétérocycle peut contenir 1 ou 2 hétéroatomes et/ou hétérogroupes identiques ou différents. Comme hétéroatomes, on indique l'atome d'oxygène, de soufre ou d'azote et comme hétérogroupe, N-alcoyle, où le radical alcoyle du radical N-alcoyle contient 1 à 4 atomes de carbone.

Comme exemple d'hétérocycle, on citera les radicaux pyrrolidine, pipéridine, pipérazine, hexaméthylèneimine, morpholine et N-méthylpipérazine.

**4.** Formulations suivant la revendication 2, dans lesquelles on met en ouvre comme agent actif a, un ou plusieurs composés de la formule (I), dans laquelle les restes ont la signification suivante :

R représente un reste le cas échéant substitué de la série des radicaux hétéroarylméthyle ou hétéroaryléthyle, où comme hétéroaryle, on cite :

les radicaux thiényle, furyle, thiazolyle, imidazolyle, pyridyle, benzthiazolyle, où les substituants sont :

les radicaux méthyle, éthyle, méthoxy, éthoxy, méthylthio, éthylthio, trifluorométhyle, hydroxyle, les atomes de fluor, de chlore et de brome, les radicaux cyano, nitro, amino ;

A représente l'atome d'hydrogène ainsi qu'un radical alcoylène bifonctionnel, le cas échéant substitué, lié au reste Z, avec 2 atomes C, où comme substituant, on citera les substituants énumérés ci-dessus et où le radical alcoylène peut être interrompu par 1 hétéroatome de la série N, O ou S ;

A et Z peuvent former ensemble, avec les atomes sur lesquels ils sont liés, un hétérocycle saturé ou insaturé à 5 ou 6 membres. L'hétérocycle peut contenir 1 ou 2 hétéroatomes et/ou hétérogroupes identiques ou différents. Comme hétéroatomes, on indique l'atome d'oxygène, de soufre ou d'azote et comme hétérogroupe, N-alcoyle, où le radical alcoyle du radical N-alcoyle contient 1 ou 2 atomes de carbone.

E représente les radicaux $NO_2$, CN.

X représente le reste -CH= ou =N-.

Z représente les restes le cas échéant substitués alcoyle, -O-R', -S-R', -NR'R' (les restes R' sont identiques ou différents), où R' et les substituants ont la signification suivante :

R' représente l'atome d'hydrogène ainsi qu'un reste le cas échéant substitué de la série des radicaux

acyle, alcoyle, aryle, aralcoyle, hétéroaryle, hétéroarylalcoyle.
Comme reste acyle, on cite les radicaux formyle, alcoylcarbonyle, arylcarbonyle, alcoylsulfonyle, arylsulfonyle, (alcoyl)(aryl)-phosphoryle.
Comme radical alcoyle, on cite un radical alcoyle en $C_{1-4}$.
Comme radical aryle, on cite le radical phényle.
Comme radical aralcoyle, on cite les radicaux phénylméthyle, phényléthyle.
Comme radical hétéroarylalcoyle, on cite les radicaux hétéroarylméthyle, hétéroaryléthyle, où comme hétéroaryle, on cite les radicaux thiényle, furyle, thiazolyle, imidazolyle, pyridyle, benzthiazolyle.
Comme substituant du reste R', on indique :

les radicaux méthyle, éthyle, méthoxy, éthoxy, méthylthio, éthylthio, halogénoalcoyle avec 1 ou 2 atomes de carbone et 1 à 5 atomes d'halogène, où les atomes d'halogène sont identiques ou différents et sont les atomes de fluor, de chlore ou de brome, le radical hydroxyle, les atomes de fluor, de chlore et de brome, les radicaux cyano, nitro, amino, monoalcoyl- et dialcoylamino avec de préférence, 1 ou 2 atomes de carbone par radical alcoyle, les radicaux carboxyle, carbalcoxy avec 2 ou 3 atomes de carbone, les radicaux sulfo ($-SO_3H$), alcoylsulfonyle avec 1 ou 2 atomes de carbone, phénylsulfonyle, chloropyridylamino et chloropyridylméthylamino.

**5.** Formulations suivant la revendication 1, dans lesquelles comme agent actif a, sont présents un ou plusieurs des composés des formules générales (II), (III) et (IV) :

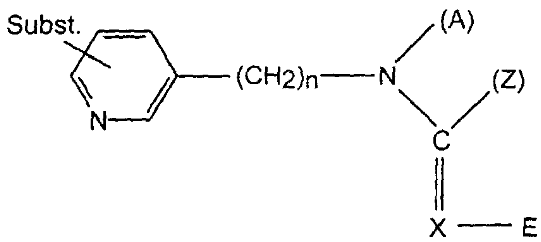

(II)

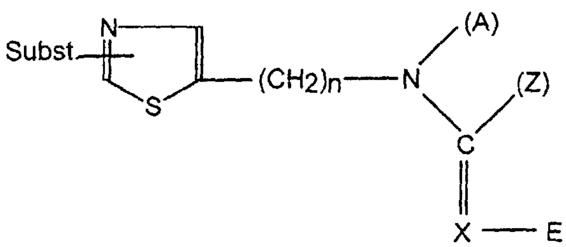

(III)

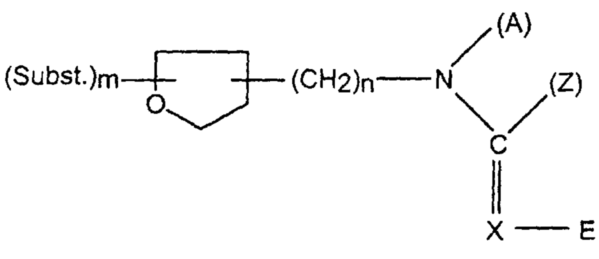

(IV)

dans lesquelles :

n représente 1 ou 2 ;
m représente 0, 1 ou 2 ;
Subst. représente l'un des substituants indiqués aux revendications 3 et 4, et
A, Z, X et E ont les significations indiquées aux revendications 2 à 4.

**6.** Formulations suivant la revendication 1, dans lesquelles comme agent actif a, sont présents un ou plusieurs des composés suivants :

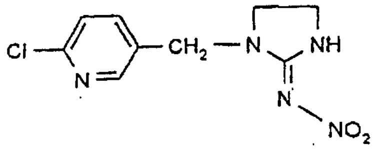

Imidacloprid

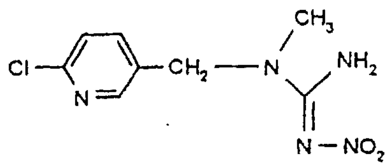

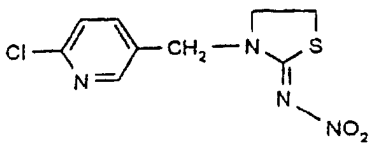

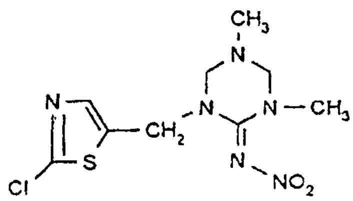

AKD 1022

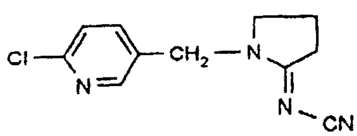

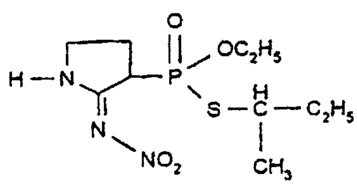

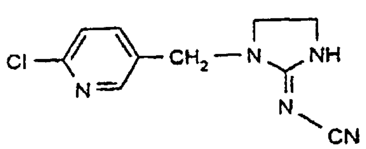

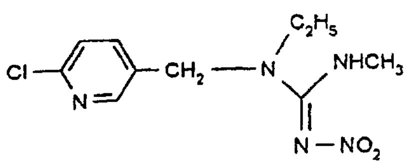

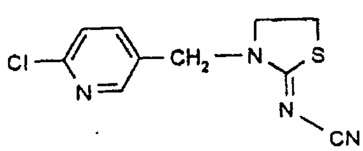

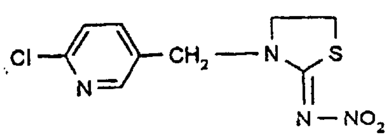

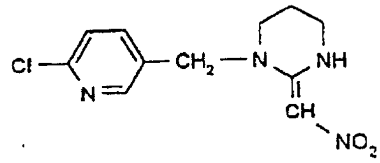

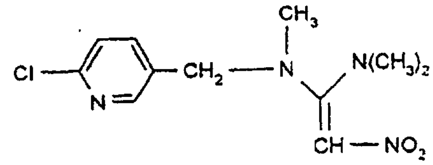

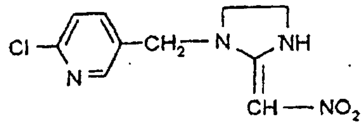

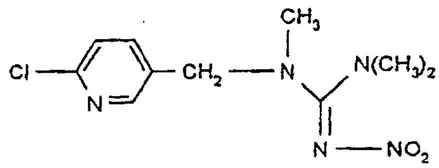

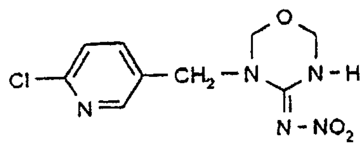

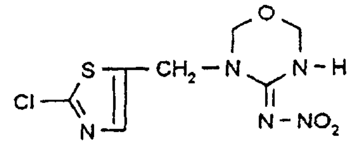

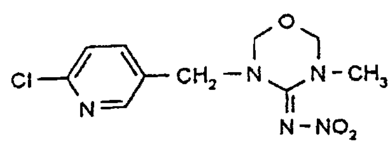

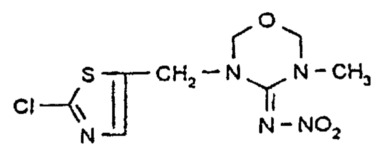

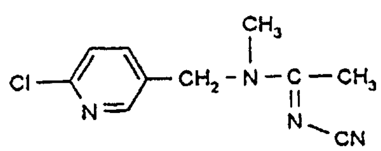

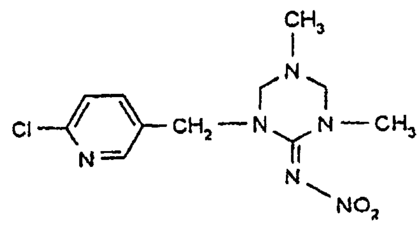

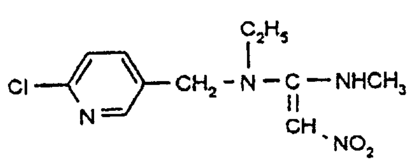

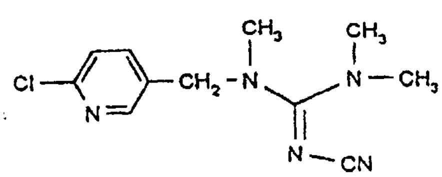

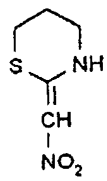

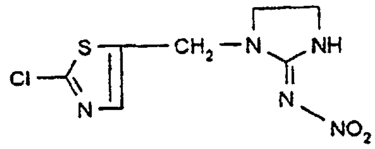

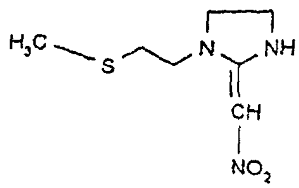

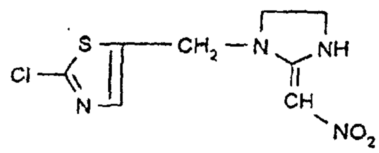

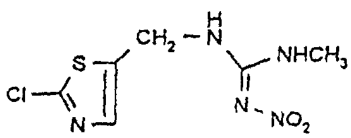

Ti435

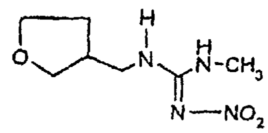

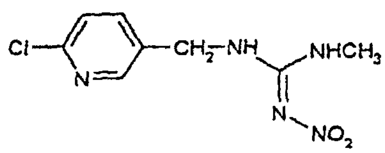

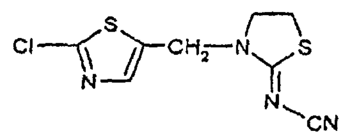

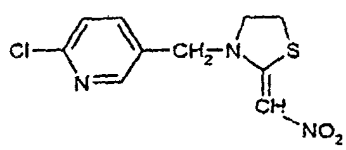

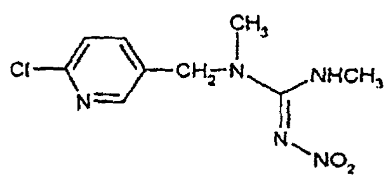

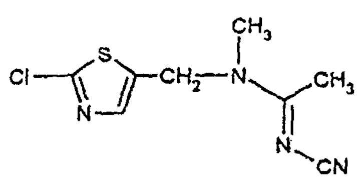

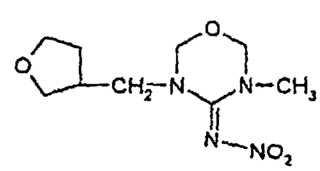

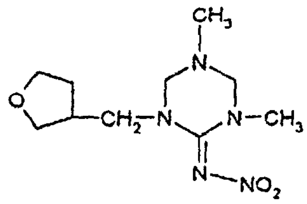

7. Procédé de préparation de l'agent suivant la revendication 1, **caractérisé en ce que** l'on mélange le ou les agents actifs avec l'eau et le ou les solvants indiqués en une solution homogène et le cas échéant, on ajoute les autres auxiliaires.